# EUROPÄISCHE PATENTSCHRIFT

(11) **EP 2 637 743 B1**
(45) Veröffentlichungstag und Bekanntmachung des Hinweises auf die Patenterteilung: **10.01.2018**
(21) Anmeldenummer: 11793358.0
(22) Anmeldetag: 10.11.2011
(51) Int. Cl.: A61N 5/06

(54) **OPTISCHES BESTRAHLUNGSGERÄT FÜR DERMATOLOGIE UND KOSMETIK**
OPTICAL IRRADIATION APPLIANCE FOR DERMATOLOGY AND BEAUTY CARE
APPAREIL D'IRRADIATION OPTIQUE POUR LA DERMATOLOGIE ET LA COSMÉTIQUE

(30) Priorität: 15.03.2011 DE 102011013988; 17.01.2011 DE 102011008705; 10.11.2010 WO PCT/EP2010/067192
(43) Veröffentlichungstag der Anmeldung: 18.09.2013
(73) Patentinhaber: Nath, Günther, 82031 Grünwald (DE)
(72) Erfinder: Nath, Günther, 82031 Grünwald (DE)
(74) Vertreter: Glück Kritzenberger Patentanwälte PartGmbB
(86) Internationale Anmeldenummer: PCT/EP2011/069888
(87) Internationale Veröffentlichungsnummer: WO 2012/062884

(56) Entgegenhaltungen:
- EP-A1- 1 736 204
- EP-A1- 2 008 688
- EP-A2- 1 430 850
- WO-A1-99/58195
- WO-A1-2008/058713
- WO-A1-2009/018529
- WO-A1-2010/068317
- DE-A1-102005 042 268
- US-A- 3 527 932
- US-A1- 2009 254 154
- US-A1- 2009 254 155

## Beschreibung

### Technisches Gebiet

Die vorliegende Erfindung betrifft Verbesserungen eines Gerät zur dermatologischen oder kosmetischen Behandlung eines Patienten. Insbesondere soll die Wirksamkeit dermatologischer oder kosmetischer Substanzen zum Auftragen auf die menschliche Haut durch optische und mechanische Hilfsmittel gesteigert werden.

### Stand der Technik

Die vorliegende Erfindung stellt eine Weiterentwicklung meines in der PCT/ EP2010/067192 beschriebenen dermatologischen Bestrahlungsgeräts dar. Der Schwerpunkt der vorangegangenen PCT-Anmeldung liegt auf dem Gebiet der Behandlung von Nagelpilz durch das Zusammenwirken von optischer Strahlung und einer peroxydhaltigen Paste. Infolge von optisch induzierter Radikalbildung findet eine Aufweichung von pilzdurchsetzter Nagelsubstanz innerhalb von wenigen Minuten statt. Dieser Effekt ist hilfreich für die Beseitigung des pilzbefallenen Nagelmaterials.

Ein Bestrahlungsgerät gemäß dem Oberbegriff des Anspruchs 1 ist aus der WO 2008/58713 A1 bekannt.

### Beschreibung der Erfindung

Die gegenwärtige Erfindung hat zur Aufgabe, den Anwendungsbereich der in der PCT/EP2010/067192 beschriebenen Technik auf andere Indikationen der Dermatologie und der Kosmetik auszuweiten und entsprechende technische Anpassungen aufzuzeigen.
Insbesondere besteht die Aufgabe darin, mit Hilfe von intensivem Licht Wirkstoffe, welche in dermatologisch verwendeten Salben, Gelen oder Pasten enthalten sind, schneller und tiefer in die obersten Hautschichten einzuschleusen um somit eine bessere Wirkung zu erzielen. Als Beispiel sei hier die Einschleusung entzündungshemmender oder schmerzstillender Gele, Salben oder Pasten in die Haut mit wirksamer Unterstützung intensiver optischer Strahlung bei gleichzeitigem flächigen Andruck an das Gewebe genannt.
Voltaren® Schmerzgel oder Aloe Vera Gel® oder heparinhaltige Geie sind nur drei konkrete Beispiele von vielen dermatologischen Gelen, Salben oder Tinkturen, die sich für die optische Einschleusung in die Haut sehr gut eignen.
Eine weitere Anwendung des Bestrahlungsgerätes der vorliegenden Erfindung besteht auf dem Gebiet der Kosmetik. Hier geht es vor allem darum, die Glättung von Falten in der Haut durch kombinierte Einwirkung optischer Strahlung und konventioneller kosmetischer Gele, Salben oder Flüssigkeiten und flächigem Andruck auf das Gewebe zu verbessern. Als Beispiel sei hier die bessere und schnellere Einschleusung Hyaluron - haltiger Gele, Salben oder Flüssigkeiten in die Haut mit Hilfe intensiver optischer Strahlung und flächigem Gewebeandruck angeführt.

Im Einzelnen soll das erfindungsgemäße Bestrahlungsgerät folgende Aufgaben erfüllen;
1.) Es soll klein und leicht sein und von Hand geführt werden können.
2.) Es soll trotz seiner Kleinheit und Handlichkeit eine hohe Strahlleistungsdichte von mindestens ca. 50 - 100 mW/cm<2> auf einem Gewebeareal von etwa 0,5 - 3 cm im Durchmesser erzeugen, so dass man nach maximal 30 Sekunden auf der Haut schon eine Wärmewirkung verspürt.
3.) Das Bestrahlungsgerät soll im allgemeinen mit dem zu bestrahlenden Area! über einen Gewebeandruckkörper (im folgenden auch Aufsatzelement oder Kappe genannt) kontaktieren können um auf diese Weise immer eine reproduzierbar gleiche Strahlleistungsdichte am Gewebe zu erzeugen.
4.) Der Gewebeandruckkörper muss hochtransparent sein. Er sollte weiterhin so weich und flexibel sein, dass er sich bei leichtem Druck auf das Gewebe den dort vorherrschenden Unebenheiten anpasst.
5.) Der Gewebeandruckkörper muss leicht und schnell auswechsel- oder abnehmbar sein und soll für den bestimmungsgemäßen Gebrauch medizinisch zugelassen sein. Darüber hinaus soll er gegenüber den medizinisch und kosmetisch verwendeten Gels, Salben und Flüssigkeiten chemisch inert sein und er muss desinfizierbar sein mit den in der Medizin bzw. Kosmetik gebräuchlichen flüssigen Desinfektionsmitteln (z. B. Cidex®, Mucocid®, Gigasept®, u. a.). Noch besser: der Gewebeandruckkörper sollte autoklavierbar sein. Er sollte auch spülmaschinentauglich sein.
6.) Der Gewebeandrückkörper sollte eine Vorrichtung aufweisen, die es erlaubt die in der Dermatologie und/oder Kosmetik gebräuchlichen wirkstoffhaltigen Gels bzw. Salben äußerst sparsam und verlustfrei einzusetzen und anzuwenden, weil diese meistens sehr teuer sind. So kostet z. B. ein Hyaluron®-haltiges Gel, welches in der Kosmetik zum Glätten von Falten verwendet wird, bereits 1€ pro ml, wobei es sich hier noch um eine niedrigere Konzentration des Wirkstoffs und ein nicht besonders hochwertiges Gel handelt.
7.) Das Bestrahlungsgerät sollte gefahrlos im Gesicht und dort auch in dem Bereich um die Augen verwendbar sein, ohne dass vagabundierende Streustrahlung ins Innere des Auges gelangen kann und dort eine Blendwirkung oder Schädigung verursacht.

Diese Aufgaben werden wenigstens teilweise durch das in Anspruch 1 definierte Behandlungsgerät gelöst. Die Unteransprüche 2 bis 22 betreffen bevorzugte Ausführungsbeispiele.

Gemäß einem weiteren Aspekt der vorliegenden Erfindung, die sich insbesondere für dermatologische und forensische Untersuchungen eignet, soll ein Bestrahlungsgerät geschaffen werden, mittels dem sich Wellenlänge und Intensität des emittierten Lichts für spezielle optische Untersuchungen optimieren lassen.
Gemäß einem weiteren Aspekt der vorliegenden Erfindung, die sich insbesondere für das Dentalbleaching eignet, soll ein Bestrahlungsgerät geschaffen werden, das den optischen Strahlungsquerschnitt des emittierten Lichts möglichst optimal an den zu behandelnden Körperbereich anpasst.
Gemäß einem Ausführungsbeispiel der Erfindung werden die Aufgaben wie folgt gelöst:

Das optische Bestrahlungsgerät enthält als Lichtquelle eine oder mehrere (Array) Lumineszenzdioden in möglichst enger räumlicher Anordnung um maximale Leuchtdichte zu erzeugen. Bewährt hat sich eine quadratische Anordnung von vier Einzeldioden oder eine rechteckige Anordnung von 6 Einzeldioden. Insgesamt können sich bis zu 12 Einzeldioden in angenähert kreisförmiger Anordnung auf einer Fläche von maximal 9 - 10 mm im Durchmesser befinden. Der Spektralbereich, innerhalb dessen die LEDs emittieren, kann im Bereich von 350 nm < λ < 1000 nm liegen. Die elektrische Aufnahmeleistung des Dioden-Arrays kann zwischen 5 Watt und 40 Watt liegen.

Das Dioden-Array ist gut wärmeleitend mit einem metallischen Kühlkörper verbunden, welcher gleichzeitig als länglicher Handgriff ausgebildet sein kann oder welcher in einem länglichen Handgriff enthalten sein kann. Der Handgriff besitzt eine (oder mehrere) Lufteintritts- und Luftaustrittsöffnung(en). Ein Lüfter mit einer Kühlleistung von etwa 80 l/min sorgt für die Wärmeabgabe des Kühlkörpers.

Eine bevorzugte Konfiguration ist ein LED-Array mit vier Einzeldioden in Serienschaltung in quadratischer oder sternförmiger Anordnung mit einer elektrischen Aufnahmeleitung von ca. 10 - 15 Watt (15 Volt, 1 Ampere).

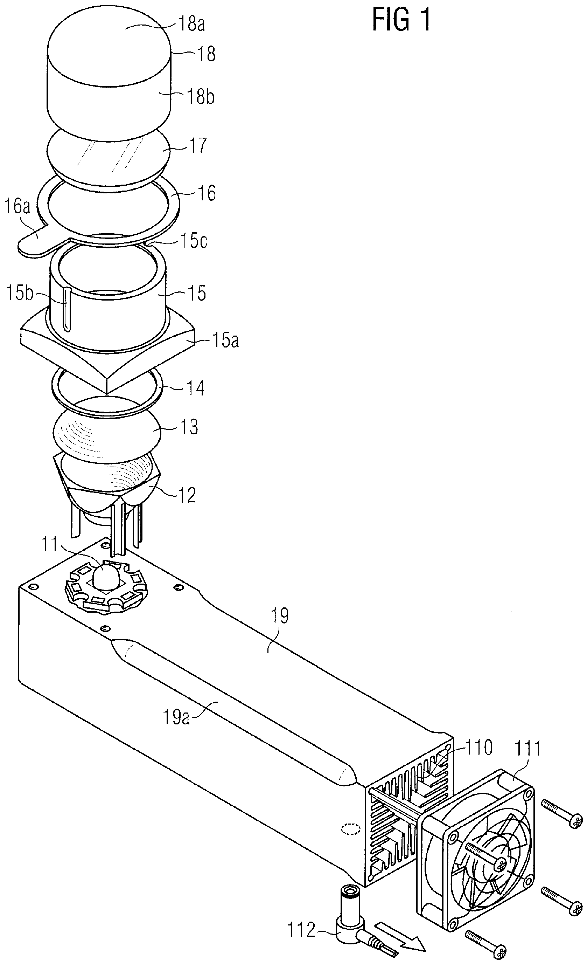

In dieser Ausführung des Bestrahlungsgeräts, wie es in Figur 1 dargestellt ist, kann man im Dauerbetrieb arbeiten. Dabei erwärmt sich das Handstück aus Aluminium zwar, doch hält sich die Temperaturerhöhung noch im tolerierbaren

Bereich und die Emissionsleistung der vier LEDs bleibt auch bei Dauerbetrieb konstant. Bei Optimierung der Kühlung der LEDs und des von Hand zu führenden Griffes liegt die obere Grenze der verwendbaren elektrischen Aufnahmeleistung des Dioden-Arrays bei ca. 30 Watt, vorausgesetzt, man will die Kleinheit und Handlichkeit des Bestrahlungsgerätes nicht aufgeben.

Die Strahlung des LED-Arrays wird senkrecht zur Griffachse emittiert, wobei ein trichterförmiger Reflektor und noch eine Sammellinse oder eine Planplatte die sich im Inneren eines Tubusrohres befinden, die Strahlung bündeln und das LED-Array nach außen hin einschließen und absichern gegen Kontaminierung.

Über das Tubusrohr wird der Gewebeandruckkörper meist in Form einer Kappe mit engem Sitz übergestülpt, wobei die Kappe mit ihrer Strahlaustrittsfläche aus einem Kunststoff-Polymer besteht, welches zumindest transluzent, vorzugsweise aber hochtransparent ist. Als Material für die Kappe bzw. die Gewebeandruckfläche kann in erster Linie Silikonkautschuk aber auch Materialien wie FluorKohlenstoff Polymere (Teflon® FEP, Teflon® MFA, Teflon® PTFE, Hyflon® THV) oder Polyurethan oder Polyethylen (auch vernetzt) Verwendung finden.

Bevorzugt sind elastomere Kunststoffe, welche sehr weich und hochtransparent, chemisch weitestgehend inert und hochtemperaturbeständig (> 150°C) sind. Unter diesen sind zylindrische additionsvernetzte elastomere Silikonkautschuk-Materialien, so genannte Zwei-Komponenten Silikon Kautschuk Materialien, Zwei-Komponenten Polymethylsilikone mit Platin Katalysator wie z. B. Elastosil® RT 601 A/B als Materialien für den Gewebeandruckkörper bevorzugt.

Der Gewebeandruckkörper hat im allgemeinen die Form einer Kappe (siehe Figur 1, Bezugszeichen18) und besteht z. B. aus einem gegossenen Zwei-komponenten Silikon-Kautschuk dessen Shore A Härte bei ca. 45 liegt und dessen Reißdehnung bei etwa 100% liegt. Der Gewebeandruckkörper, z. B. in Form einer homogen geformten Kappe (Figur 1, 18), hat vorzugsweise die Eigenschaft, dass man zwei verschieden geformte Geometrien der Strahlaustritts- bzw. der Gewebeandruckfläche (Figur 1, 18a) einstellen kann, und zwar auf reversible Weise.

Die eine Geometrie ist konkav und dient der Aufnahme eines Gels oder einer Salbe. Mit dieser Geometrie kann man das Gel (Salbe) auf der Gewebeoberfläche materialsparend verteilen. Die zweite Geometrie ist plan oder leicht konvex. Mit dieser Geometrie lässt sich das Gel (Salbe) mit dem Wirkstoff, in das Gewebe einmassieren bzw. einschleusen bei gleichzeitiger Bestrahlung des Gewebes mit intensivem Licht.

Da eine bevorzugte verwendete Lichtbestrahlung im Spektralbereich zwischen 445 nm und 485 nm liegt, die eine außergewöhnlich hohe Leistungsdichte von z. B. 400 mW/cm² am Gewebe erzeugt, findet fast instantan eine merkliche Temperaturerhöhung in den obersten Hautschichten (Epidermis, Dermis) statt. Dadurch bildet sich ein leichtes Wärmeerythem aus, so dass das wirkstoffhaltige Gel (Salbe) schneller und tiefer in das Gewebe eingeschleust werden kann. Das Wärmeerythem, eine leicht rötliche Verfärbung der Haut, bildet sich ca, 10 Minuten nach der Lichtbestrahlung wieder zurück.

Der dosierte Andruck der Kappe an das zu behandelnde Gewebe bewirkt dort außerdem eine verminderte Durchblutung, die ein tieferes Eindringen der Strahlung zur Folge hat, weil Blut ein starker Lichtabsorber ist. Dadurch ist eine optische Behandlung tieferer Gewebeschichten mit oder ohne wirkstoffhaltige Substanzen möglich.

Ferner können statt oder zusätzlich zu den wirkstoffhaltigen Substanzen auch bestimmte Fluide als Gleit- und/oder Immersionsmittel auf die Kappe aufgetragen werden, um bei der Behandlung störende Reibung zwischen dem Kappenmaterial und der Haut zu vermeiden und/oder eine verbesserte optische Einkopplung des Lichts in das Gewebe zu erzielen.

### Kurze Beschreibung der Zeichnungen

Im Folgenden wird das erfindungsgemäße Bestrahlungsgerät anhand der Figuren 1 bis 6 und anhand konkreter Ausführungsbeispiele näher erläutert. Darin zeigt bzw. zeigen:
Figur 1 eine Explosionsdarstellung des Behandlungsgeräts gemäß einem ersten Ausführungsbeispiel der Erfindung;
Figuren 2a und 2b Querschnittsansichten des Behandlungsgeräts gemäß dem ersten Ausführungsbeispiel zur Erläuterung der erfindungsgemäßen Verwendung;
Figuren 3a und 3b weitere Querschnittsansichten des Behandlungsgeräts gemäß dem ersten Ausführungsbeispiel zur Erläuterung der erfindungsgemäßen Verwendung;
Figur 4a eine perspektivische Ansicht des Behandlungsgeräts gemäß dem ersten Ausführungsbeispiel der Erfindung;
Figur 4b eine Querschnittsansicht des Behandlungsgeräts gemäß dem ersten Ausführungsbeispiel der Erfindung;
Figur 4c eine Querschnittsansicht des Gewebeandruckkörpers des Behandlungsgeräts gemäß einem zweiten Ausführungsbeispiel der Erfindung;
Figur 5a eine perspektivische Ansicht des Behandlungsgeräts gemäß einem dritten Ausführungsbeispiel der Erfindung;
Figur 5b eine perspektivische Ansicht des Behandlungsgeräts gemäß einem vierten Ausführungsbeispiel der Erfindung;
Figur 6a eine Querschnittsansicht des Behandlungsgeräts gemäß einem fünften Ausführungsbeispiel der Erfindung;
Figur 6b eine Querschnittsansicht des Behandlungsgeräts gemäß einem sechsten Ausführungsbeispiel der Erfindung;
Figur 6c eine Querschnittsansicht des Behandlungsgeräts gemäß einem siebten Ausführungsbeispiel der Erfindung;
Figur 6d eine Querschnittsansicht des Gewebeandruckkörpers des Behandlungsgeräts gemäß einem achten Ausführungsbeispiel der Erfindung; und
Figur 6e eine perspektivische Ansicht des Gewebeandruckkörpers des Behandlungsgeräts gemäß einem neunten Ausführungsbeispiel der Erfindung.

### Detaillierte Beschreibung mehrerer Ausführungsbeispiele

Figur 1 zeigt schematisch in Form einer Explosionszeichnung den Aufbau des erfindungsgemäßen dermatologischen Bestrahlungsgerätes.

Die Strahlungsquelle (11) enthält vorzugsweise eine oder mehrere Lumineszenzdioden (LEDs). Besonders bevorzugt ist ein Dioden-Array welches sich aus 4 oder 6 oder noch mehr LEDs zusammensetzt die meistens in Serie geschaltet sind. Bei Akkubetrieb kann man die LEDs aber auch paarweise parallel miteinander verschalten. Die elektrische Gesamtleistung des Dioden-Arrays liegt zwischen 5 und 25 Watt bevorzugterweise im Bereich von 5 - 20 Watt, besonders bevorzugt im Leistungsbereich zwischen 8 und 18 Watt.

Die Dioden des Arrays können im Spektralbereich zwischen 320 nm und 150 nm, vorzugsweise zwischen 350 nm und 1000 nm, emittieren. Die Dioden können alle in einem einheitlichen Spektralbereich emittieren oder gemischt in zwei verschiedenen Spektralbereichen.

So kann man zum Beispiel ein Array bestehend aus 4 Einzeldioden verwenden wobei 2 Dioden im Rotbereich und 2 Dioden im Blaubereich emittieren. Man kann auch 2 Dioden im Blaubereich und 2 Dioden im UVA-Bereich miteinander kombinieren. Die Wahl der Spektralbereiche der Dioden hängt davon ab ob man eine größere Eindringtiefe im Gewebe (rot, nahes infrarot) oder eine geringere Eindringtiefe (blau, violett) oder auch noch eine photochemische Wirkung durch Radikalbildung (UVA, violett) erreichen will.

Das Dioden-Array (11) ist gut wärmeleitend mit einem länglichen Kühlkörper (19) aus Aluminium mit nach innen gerichteten Kühllamellen (110) verbunden, der hier gleichzeitig auch als Griff des von Hand zu führenden Bestrahlungsgerätes dient. Der Kühlkörper-Griff (19) hat eine Länge von ca. 120 - 140 mm und einen rechteckigen Querschnitt von 30 x 30 mm. Die 4 Längskanten (19a) des Kühlkörpers (19) sind verrundet so dass man ihn bequem in der Hand halten kann.

Der Lüfter (111) sorgt für den nötigen Luftdurchsatz zur Kühlung des Dioden-Arrays (11) und des Handgriffs (19) weil die elektrische Verlustleistung immerhin bei ca. 12 - 15 Watt liegen kann.

Die elektrische Versorgungsspannung, die im medizinisch unbedenklichen Kleinspannungsbereich liegt (typisch 15 - 24 V), wird durch den Koaxialstecker (112) besorgt, und wird im einfachsten Falle von einem preisgünstigen Stecknetzteil geliefert (Typisch 15V dc, 1 A maximal).

Für die rationelle Montage ist es von Vorteil, wenn am lüfterseitigen Ende des Handgriffs (19) ein Teil der innenliegenden Kühllamellen (110) auf eine Tiefe von ca. 2 cm ausgefräst sind (hier nicht dargestellt), so dass die Verdrahtung des Dioden-Arrays (11) mit der Buchse des Steckers (112) und die Unterbringung der für das Array und den Lüfter erforderlichen Vorschaltwiderstände und evtl. noch anderer elektronischer Bauelemente bequem durchzuführen sind.

Die Strahlung, welche von der Lumineszenzdiode (11) bzw. dem Array mehrerer solcher Dioden emittiert wird, gelangt zunächst in einen kegelförmigen Reflektor (12) mit innen hochverspiegelter Wandung. Der Reflektor (12) kann auch parabolische Symmetrie aufweisen, wobei sich die über dem Array angebrachte Glaskuppel (Dom) in etwa im Brennpunkt des Reflektor-Paraboloids befindet.

Unmittelbar darauf folgend befindet sich eine Linse (13) aus Glas oder Plexiglas die hier zum Beispiel eine Plankonvex-Linse mit einer Brennweite von 3 - 10 cm sein kann. Statt einer Linse kann man auch eine optische Planplatte verwenden, insbesondere dann, wenn der Reflektor (12) ein parabolischer Reflektor ist. Reflektor (12) und Linse (13) können auch wegfallen wenn die Innenfläche des Tubus (15) hochverspiegelt ist.

Linse (13), Reflektor (12) und Dioden-Array (11) befinden sich im Inneren eines rohrförmigen Tubus (15) mit Sockelplatte (15a), die in diesem Beispiel als ein homogenes Aluminiumteil ausgebildet sind. Die Linse (13) kann beidseitig gelagert sein, einmal auf dem Rand des Reflektors (12) und andererseits durch einen hier nicht sichtbaren Vorsprung im Inneren des Tubus (15), wobei ein O-Ring (14) aus einem elastomeren Material zwischen Linse (13) und dem Vorsprung im Innenraum des Tubus (15) positioniert sein kann, um die Linse gegen mechanische Erschütterungen zu schützen.

In der äußeren Umfangsfläche des Rohres (15) befindet sich eine senkrecht nach unten verlaufende Rille (15b), die am oberen Rand des Rohres (15) beginnt aber nicht unbedingt bis auf das Niveau des Sockels (15a) verläuft. Sie kann 1 - 2 mm vorher enden. Die Rille (15b) ist ca. 1 - 2 mm breit und nur wenige 1/10 mm tief. Eine weitere Rille (15c) gleicher Geometrie kann sich, um 180° versetzt, ebenfalls auf der Umfangsfläche des Rohres (15) befinden.

Ein metallischer Ring (16) mit Lasche (16a) wird über das Rohr (15) gestülpt und lagert auf dem Sockel (15a). Der Innendurchmesser des Ringes (16) ist um mehrere 1/10 mm größer als der Außendurchmesser des Rohres (15), so dass der Ring durch manuellen Druck auf die Lasche (16a) etwas gekippt werden kann. Die Position der Lasche (16a) befindet sich bevorzugt direkt unterhalb der Rille (15b) oder um 180° versetzt zur Rille (15b).

Über das Rohr (15) wird bei den meisten Anwendungen des dermatologischen Bestrahlungsgerätes eine Kappe (18) aus hochtransparentem Silikon-Kautschuk gestülpt, wobei die Mantelfläche (18b) der Kappe (18) bis auf Kontakt mit dem Sockel (15a) bzw bis auf Kontakt mit dem Ring (16) geschoben wird. In dieser Position der Kappe (18) soll die Rille (15b, 15c) noch um mindestens ca. 1 mm von der Mantelfläche (18b) der Kappe (18) überdeckt werden. Da die Silikonkappe (18) straff auf dem Tubus (15) aufsitzt, wird am Tubus (15) außen eine dünne Schicht eines Fettes oder eines hochviskosen Öls (Silikonfett, Silikonöl, Vakuumfett, Vaseline, etc.) aufgetragen, so dass man die Kappe (18) leicht überstülpen und auswechseln kann, wobei im Falle der Überdeckung der Rille(n) (15b, 15c) durch die Kappe (18) ein luftdichter Abschluss zum innenraum des Tubus (15) bestehend aus dem Volumen zwischen Linse (13) und der Lichtaustrittsfläche (18a) der Kappe (18) entsteht.

Vorausgesetzt dass auch die Linse (13) luftdicht innerhalb des Tubus (15) eingeklebt oder eingepresst ist, ergibt sich so ein luftdichtes Volumen im inneren des Tubus (15) zwischen Linse (13) und der konvex oder schwach konvex nach außen gewölbten Lichtaustrittsfläche (18a) der Kappe (18). Unter der Voraussetzung geeigneter Dimensionierung der Wandstärke und des Innendurchmessers der Kappe (18) sowie bei geeigneten Werten der Shore A Härte sowie der Elastizitätswerte (Dehnbarkeit) des hochtransparenten additionsvernetzten Silikonkautschuks kann man leicht die konvexe Oberfläche (18a) der Kappe (18) durch Druck mit dem Finger oder dem Daumen oder mit Hilfe eines geeigneten Formwerkzeuges in eine konkave, nach innen gewölbte Oberfläche (18a) der Kappe (18) verwandeln, die dann infolge des entstehenden Unterdrucks in dem Raum definiert durch die Linse (13) und die Lichtaustrittsfläche (18a) der Kappe (18) auch so bestehen bleibt. Dieser Unterdruck entsteht dadurch, dass einerseits die Luft aus dem Volumen unterhalb der Kappenfläche (18a) herausgepresst wird und andererseits durch die Rückstellkraft der verformten Kappenfläche (18a).

Drückt man jetzt die Lasche (16a) des metallischen Ringes ein wenig nach oben (oder unten), so dass die Kappe (18) einseitig um ca. 2 mm angehoben wird, so gelangt Luft in eine der Rillen (15b oder 15c) und die konkave Oberfläche der Kappe (18) verwandelt sich wieder in eine konvexe Oberfläche (18a) zurück.

Die konkave, nach innen gewölbte Oberfläche (18a) der Kappe (18) bildet eine Mulde, welche sich sehr gut zur Aufnahme der minimal nötigen Menge eines wirkstoffhaltigen Gels (Creme, Paste, Flüssigkeit) eignet, wobei das Verreiben und Einmassieren des Gels durch kreisende Bewegung und leichten Andruck der Kappe (18) auf das Gewebe erfolgt. Dieses geschieht unter gleichzeitiger Bestrahlung mit dem Licht der LEDs. Wenn das Gel (Creme, Paste, Flüssigkeit) auf der Haut verteilt ist, kann durch Druck auf die Lasche (16a) des ringförmigen Belüftungshebels die Mulde der Kappe (18) wieder in eine konvexe (oder plane) Oberfläche zurückverwandelt werden, die sich besser eignet für die weitere Verteilung und Einreibung des Gels bei gleichzeitiger Bestrahlung des Gewebes mit dem LED-Licht.

Wenn die Schichtdicke d der Lichtaustrittsfläche (18a) der Kappe (18) nicht größer als 1-3 mm ist, und wenn die Shore-A Härte des additionsvernetzten hochtransparenten Silikonkautschuks kleiner als 90, vorzugsweise ca. 50, ist und wenn schließlich die aufgestülpte Kappe (18) noch etwas (z. B. 0,5 - 15 mm) über den Rand des Tubus (15) hinausragt, so dass man beim Andruck der Kappe an das Gewebe nicht den harten Rand des metallischen Tubus (15) spürt, erhält man so ein weiches flexibles Gewebeandruckelement, welches sich bei leichtem Andruck wie ein Kissen an alle Unebenheiten des Gewebes anschmiegt und so für eine gleichmäßige optische Verteilung und Einschleusung des wirkstoffhaltigen Gels (Salbe, Paste, Creme, Flüssigkeit) in das Gewebe sorgt.

Das Plättchen (17), welches sich im Strahlengang befindet, dient als Wellenlängenschieber und kann wegen fluoreszierender Leuchtstoffe, welche darin enthalten sind, optimal verwendet werden, wenn man mit dem gleichen Bestrahlungsgerät und der gleichen LED (bzw. LED-Array) eine längerwellige tiefer in das Gewebe eindringende Strahlung erzeugen will.

Um die Blendwirkung der aus der Andruckkappe (18) austretenden LED-Strahlung, die besonders störend ist im Wellenlängenbereich um 460 nm oder im längerwelligen Spektralbereich, zu reduzieren, kann man folgende Maßnahmen treffen: Die LED soll nur dann leuchten wenn die Andruckkappe (18) Gewebekontakt hat, wobei ein definierter minimaler Andruck der Kappe (18) auf das Gewebe sich auf einen Mikrotaster überträgt, der seinerseits den LED-Strom schaltet. Dieser Mechanismus lässt sich z. B. realisieren indem der Tubus (15) von Figur 1 im Inneren, konzentrisch liegend, einen zweiten, dünnwandigen Tubus enthält, der eine minimale leichtgängige Hubbewegung von nur wenigen Millimetern innerhalb des ersten äußeren Tubus (15) ausführen kann und welcher auf seiner Lichtaustrittsöffnung die Gewebeandruckkappe (18) trägt.

Der innere bewegliche Tubus ragt aus dem äußeren Führungstubus (15) um ca. 1 - 2 cm heraus, so dass man auf diesen die Gewebeandruckkappe überstülpen kann. Der untere Rand des inneren Tubus hat mechanischen Kontakt mit dem Auslösestift (Hebel) eines Mikrotasters, welcher sich auf dem Handstück (19) des Bestrahlungsgerätes von Figur 1 befindet.

Durch die Hubbewegung des inneren Tubus bei Gewebeandruck der Kappe wird der Schaltmechanismus des Mikrotasters ausgelöst und die LED leuchtet. Beim Abheben der Andruckkappe vom Gewebe genügt meist die Rückstellkraft des Mikrotasters, die man aber noch durch zusätzliche Anbringung einer Spiralfeder verstärken kann, um den beweglichen inneren Tubus wieder hochzudrücken, wobei die LED-Lichtquelle sofort erlischt.

Alternativ kann man statt des Mikrotasters auch ein Reed-Relais als Schaltauslöser für das LED-Licht verwenden. Der innere bewegliche Tubus kann dann mit einem Permanentmagneten (z. B. einem ringförmigen Permanentmagneten) und mit einer elastischen Rückstellvorrichtung z. B. in Form einer Spiralfeder versehen sein. Bei Gewebeandruck der Kappe nähert sich der Permanentmagnet dem Reed-Relais und bewirkt bei einer Minimalentfernung von diesem die Kontaktschaltung (R ≈ 0 Ω) des Reed-Relais. Beim Abheben der Kappe (18) vom Gewebe wird der innere bewegliche Tubus durch Wirkung der Spiralfeder wieder nach außen gedrückt und das Reed-Relais schaltet in seinen Ausgangszustand (R ≈ ∞ Ω) zurück, wobei das LED-Licht erlischt.

Der Schaltmechanismus mit dem Reed-Relais ist etwas komplizierter als die Anordnung mit Mikrotaster, benötigt aber keinen definierten Schwellenwert für den Gewebandruck. Der nötige Hubweg des inneren Tubus für die Auslösung der Schaltung ist aber länger.

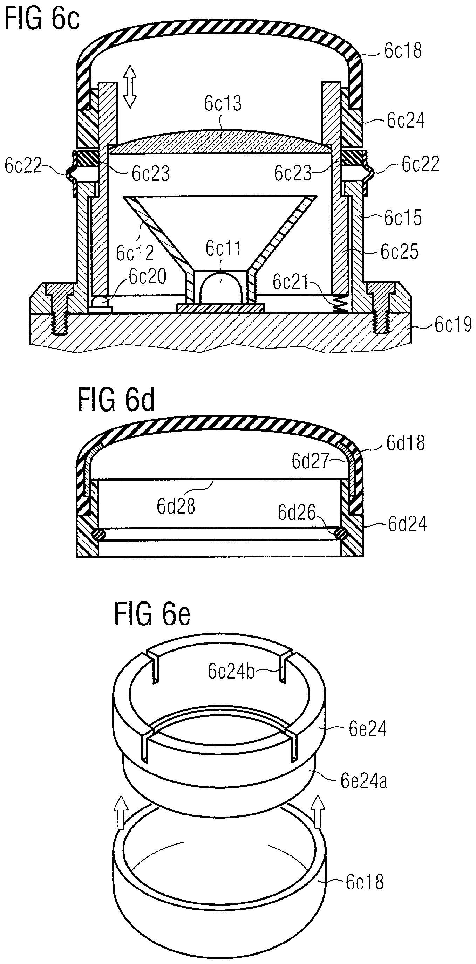

Fig. 6c veranschaulicht das Gerät, welches nur bei Gewebeandruck leuchtet. Die Andruckkappe (6c18) einschließlich des Sockels (6c24) ist hier mit einem inneren beweglichen Tubus (6c25) fest aber auswechselbar verbunden, weicher teleskopartig innerhalb eines äußeren Tubus (6c15) so gelagert ist, dass er eine kleine Hubbewegung (ca. 1 - 2 mm) innerhalb des äußeren Tubus (6c15) ausführen kann.

Die beiden Tubusrohre (6c25) und (6c15) sind ineinander verzahnt, so dass die Hubbewegung begrenzt bleibt. Bei Gewebeandruck auf die Kappe (6c18) drückt der innere Tubus (6c25) auf den Mikrotaster (6c20) und eventuell auch auf eine Spiralfeder (6c21) wodurch die LED (6c11) aktiviert wird und leuchtet. Beim Abheben des Gerätes vom Gewebe, sorgt die Rückstellkraft des Mikrotasters (6c20) und der Spiralfeder (6c21) dafür, dass der innere Tubus (6c25) in seine Ausgangslage zurück geht und die LED (6c11) erlischt.

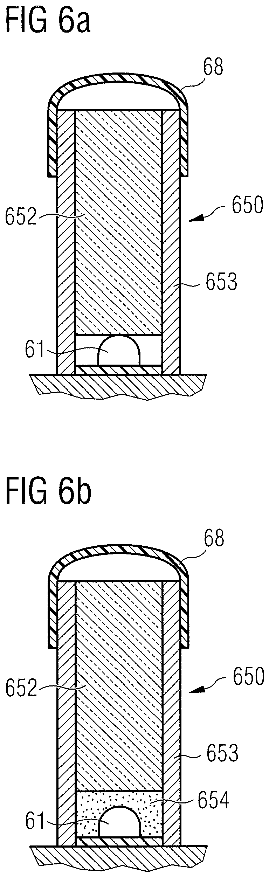

Die Silikonkappe (6c18) ist auf einen ringförmigen festen Sockel (6c24) geklebt, wodurch die rasche Auswechselbarkeit des Gewebeandruckkörpers gegeben ist. Ferner vorgesehen ist ein ringförmiger Anschlag (6c23) für den Sockel (6c24). Eine Abdichtung des Spaltes zwischen innerem und äußerem Tubus, z. B. in Form eines leicht gängigen (Mono-) Faltenbalges (6c22), verhindert das Eindringen flüssiger Substanzen in den Raum der LED (6c11), mit Reflektor (6c12) und Linse (6c13), die beide auch fehlen können, wenn der innere Tubus eine hochreflektierende Innenwandung aufweist. Alternativ kann auf den inneren beweglichen Tubus auch ein Lichtleitstab analog zu Fig 6a montiert sein dessen Lichtaustrittsfläche auf das Gewebe aufgesetzt wird, wobei die LED nur bei Gewebekontakt des Lichtleitstabes leuchtet.

Eine weitere Reduzierung der Blendwirkung der aus der hochtransparenten Silikonkappe (18) und insbesondere der aus der zylindrischen Umfangfläche (18b) austretenden Streustrahlung lässt sich durch Einfärbung der Kappe erreichen.

Man kann die Kappe so einfärben, dass die zylindrische Umfangsfläche (18b) lichtabsorbierend ist und nur die frontale Lichtaustrittsfläche (18a) hochtransparent bleibt. Es genügt auch nur den Bereich der zylindrischen Mantelfläche der Kappe lichtabsorbierend zu machen, der unmittelbar an die transparente Lichtaustrittsfläche (18a) der Kappe (18) angrenzt.

Dies lässt sich z. B. realisieren, indem man in die Gussform für die Kappe vor dem Ausgießen der Form ein zylindrisch geformtes schwarz oder lichtabsorbierend eingefärbtes Schlauchsegment einlegt welches dann in der fertig ausgehärteten Kappe integriert ist. Ein derartiges Schlauchsegment besteht z. B. aus einem abgeschnittenen Ring eines dünnwandigen schwarz eingefärbten Silikonschlauchs, der z. B. 10 mm hoch sein kann und so eingelegt wird, dass er unmittelbar an der frontalen Lichtaustrittsfläche der Kappe angrenzt.

Kombiniert man die Maßnahmen der Lichtabschaltung bei fehlendem Gewebeandruck mit der lichtabsorbierenden Eigenschaft der Kappe (18) auf zumindest einem Teil seiner Zylinderfläche (18b) so kann man meistens bei der Einarbeitung von wirkstoffhaltigen Salben oder Gelen in das Gewebe mit Hilfe von intensivem sichtbarem LED Licht auf die Verwendung von Schutzbrillen verzichten, was eine bedeutende Vereinfachung der Methode darstellt.

Die Figuren 6d und 6e veranschaulichen den gesamten, abnehm- und austauschbaren Gewebeandruckkörper bestehend aus Silikonkappe (6d18) und dem ringförmigen Sockel (6d24, 6e24) aus Kunststoff oder Metall, auf welchen die Kappe (6d18) aufgeklebt ist. Die Silikonkappe (6d18) weist auf ihrer zylindrischen inneren Wandung einen Blendschutz (6d27) auf, der zumindest in dem Bereich, welcher über den Rand des festen Sockels (6d24) hinausragt und bis in die Randzone der konvexen Lichtaustrittsfläche geht.

Wenn die Silikonkappe (6d18) noch einige Millimeter (z. B. 5 - 15 mm) über den festen Rand (6d28) des Sockels hinausragt, erreicht man eine optimale Anschmiegbarkeit der Lichtaustrittsfläche der Kappe (6d18) an die Unebenheiten der Gewebeoberfläche, welche vor allem für die Anwendungen in der Kosmetik (Faltenbehandlung) aber auch in der Medizin zur Schmerzbehandlung durch Bestrahlung von Gelenken mit Gewebekontakt eventuell auch mit gleichzeitiger Einschleusung schmerzstillender Gele oder Salben, sehr erwünscht ist.

Vorteilhaft ist hierfür, dass die Silikonkappe (6d18) dünnwandig (d ∼ 1 - 3 mm) und die Shore Härte A < 90, vorzugsweise zwischen 30 und 70 ist. Der in die zylindrische Wandung der Kappe (6d18) eingebaute Blendschutz (6d27) kann z. B. ein dünnwandiges schwarzes Band sein, welches von außen oder von innen auf die Kappe (6d18) aufgeklebt oder beim Gießen der Kappe mit eingebaut wird. Es ist auch möglich die Lichtabsorption im zylindrischen Bereich der Kappe (6d18) durch Beschichtung mit schwarz eingefärbtem Silikon innen (6d27) und/oder außen, z. B. durch "dip coating" herbeizuführen. Der O-Ring (6d26) im Sockel (6d24) sorgt für guten Sitz und Abdichtung, wenn der Gewebeandruckkörper auf den äußeren Tubus (15 Fig. 1) oder auf dem beweglichen Tubus (6c25) von Fig. 6c aufgesetzt wird.

Fig. 6e zeigt noch einmal das Ensemble von Sockel (6e24) mit Ansatz (6e24a) für die Kappe (6e18). Statt eines O-Ringes sorgen hier 4 Schlitze (6e24b) auf der Unterseite des Sockels (6e24) für festen Klemmsitz.

Die Verwendung des Sockels (6c24, 6d25, 6e24), an dem die Andruckkappe (6c18, 6d18, 6e18) oder der Lichtleitstab (650) befestigt ist und der dann auf das Zwischenstück (6c15, 6c22, 6c23, 6c25) aufgesteckt wird, ist zwar nur in den Figuren 6c bis 6e gezeigt. Gleichwohl ist dieses wichtige Element der vorliegenden Erfindung zur Verbesserung der leichten Abnehm- und Austauschbarkeit des Gewebeandruckkörpers nicht auf dieses konkrete Ausführungsbeispiel beschränkt sondern kann und soll bevorzugterweise auch in den anderen Ausführungsbeispielen verwendet werden. Insbesondere kann in den Figuren 1-4b und 5b-6b ein Sockelelement (nicht gezeigt) zwischen dem Zwischenstück (15; 25; 35; 45; 55; 550, 551; 650) und dem Aufsatzelement (18; 28; 38; 414; 418; 58; 68) angeordnet sein.

Die Beschreibung bevorzugter Ausführungsbeispiele der Erfindung wird nun anhand der Figur 1 fortgesetzt. Das Plättchen (17) kann z. B. aus Plexiglas oder einem anderen Kunststoff bestehen, welches mit einem Perylen-Farbstoff dotiert ist, insbesondere mit Perylen orange (gelb) oder Perylen rot oder, in der handelsüblichen Bezeichnung, mit Lumogen® orange (gelb) oder Lumogen® rot dotiert ist. Verwendet man die LED (11) oder das LED-Array (11) im blauen Spektralbereich, z. B. im Bereich 450 nm ≤ λ ≤ 480 nm, so liegt diese Strahlung optimal im Fluoreszenzanregungsspektrum der beiden Farbstoffe Lumogen® rot oder Lumogen® orange (gelb). Man erzeugt hierbei allerdings hoch divergente Fluoreszenzstrahlung sowohl in Vorwärts- als auch in Rückwärtsrichtung.

Drückt man das Plättchen (17), das lediglich mit der dünnen hochtransparenten Silikonschicht der Kappe (18) überzogen sein kann an das Gewebe, z.B. Hautgewebe, so wird zumindest die hoch divergente (rote, gelbe) Fluoreszenzstrahlung welche in Vorwärtsrichtung aus dem Plättchen (17) austritt im Gewebe absorbiert. Die Tatsache, dass die Fluoreszenzstrahlung hoch divergent aus dem Plättchen (17) austritt ist dabei nicht so nachteilig, weil das Gewebe selbst ein optisch stark streuendes und absorbierendes Medium darstellt. Das Fluoreszenzplättchen (17) kann auch aus Glas bestehen welches mit anderen fluoreszierenden Farbstoffen, wie z. B. mit Farbstoffen auf Basis von Zinksulfid oder Oxyden der Selten-Erd-Metalle beschichtet ist.

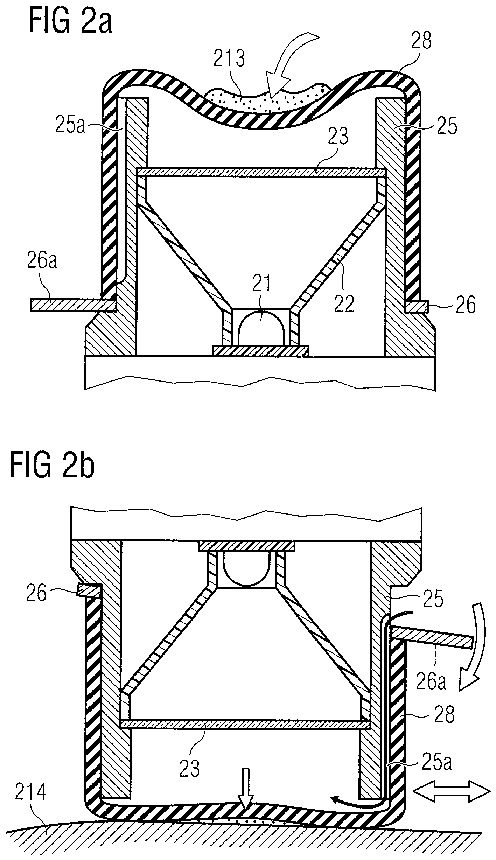

In Figur 2a und Figur 2b ist im Detail der Mechanismus der Veränderung der Geometrie (konvex-konkav) der Gewebeandruckfläche der hochtransparenten Silikonkappe (28) dargestellt.

Vor der Zugabe des wirkstoffhaltigen Gels (Creme, Paste, Salbe, Flüssigkeit) (213) wird die Kappe (28) heruntergedrückt, am besten manuell, um die in Figur 2a dargestellte Mulde zu erzeugen. Diese Mulde bleibt erhalten, weil durch das Runterdrücken die meiste Luft aus dem Raum unterhalb der Mulde bis zur optischen Platte bzw. Linse (23) herausgepresst wird, wodurch in diesem Volumen ein Unterdruck erzeugt wird. Voraussetzung ist, dass die optische Platte (Linse) (23) luftdicht in dem Tubus (25) gelagert ist. Die Lagerung erfolgt vorzugsweise durch Klebung und/oder O-Ring Dichtung.

Als nächstes wird die minimal erforderliche Menge der in die Haut einzuschleusenden wirkstoffhaltigen Substanz (213) in die Mulde gegeben, z. B. ein Hyaluron®-haltiges Gel. Daraufhin wird die Substanz auf dem betreffenden Gewebeareal verteilt durch kreisende Bewegung mit leichtem Andruck der Kappe (28) auf das Gewebe, mit oder ohne Licht. Nachdem die Substanz (213) auf dem Gewebe verteilt ist wird die Oberfläche der Kappe (28) durch Druck auf die Lasche (26a) des Ring-Hebels (26) wieder in ihre leicht konvexe oder zumindest plane Form gebracht, weil der Hebel (26, 26a) den Rand der Kappe (28) soweit verschiebt, dass Luft in die Rille (25a) eindringen kann, wodurch die Andruckfläche der Kappe (28) wieder in ihre ursprüngliche Form zurück geht. Dabei hilft natürlich die elastische Rückstellkraft der verformten Kappe.

Die weitere und eigentliche Einschleusung der wirkstoffhaltigen Substanz in das Gewebe erfolgt dann durch Andruck der wieder konvexen (planaren) Kappenoberfläche an das Gewebe (214) bei gleichzeitiger langsamer kreisender Bewegung und Lichtbestrahlung, bis Trockenheit der Gewebeoberfläche erreicht wurde.

Mit Hilfe dieser Technik kommt man mit einer minimalen Menge der Substanz (213) aus, da diese gar nicht mit den Händen der behandelnden Person in Berührung kommt und in das Silikonmaterial keinerlei Substanz einsickern kann. Dieser sparsame Verbrauch von wirkstoffhaltigem Gel (Salbe, Creme, Paste, Flüssigkeit) ist deshalb wichtig, weil derartige Substanzen (213) außerordentlich teuer sind, vor allem wenn es sich um Hyaluron®-haltige Substanzen handelt, welche bei der hier beschriebenen Erfindung eine wesentliche Rolle spielen.

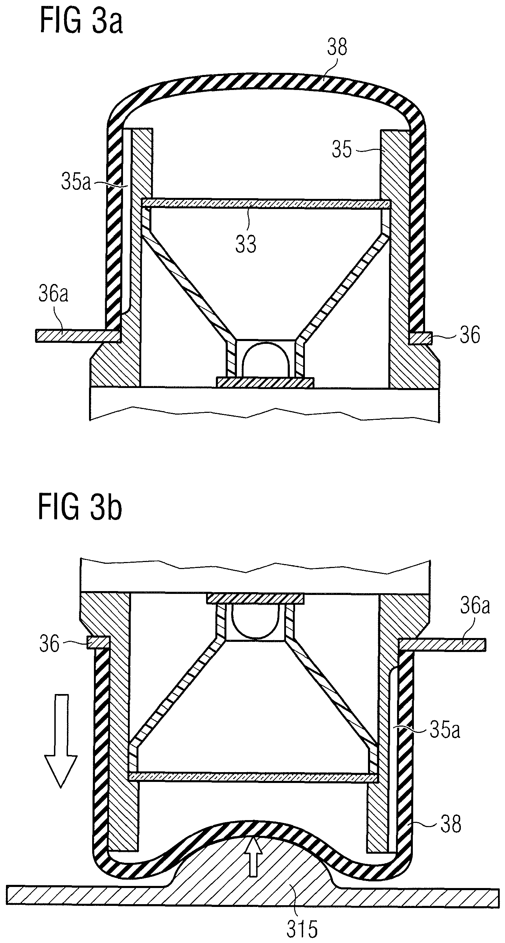

Die Figuren 3a und 3b zeigen, wie man mit Hilfe eines einfachen Werkzeuges (315) in Form einer Planplatte mit einer konvexen Erhebung die Mulde in die Kappe (38) reindrücken kann. Dabei entweicht die Luft aus dem komprimierten Volumen zwischen der Innenwand der Kappe (38) und der Außenwand des Tubus (35) nach außen. Voraussetzung ist, dass die Glasplatte (Linse) (33) im Innenraum des Tubus (35) luftdicht gelagert ist. Man kann natürlich die Mulde in die Kappe (38) auch manuell durch Finger- oder Daumendruck ohne ein Werkzeug reindrücken.

Durch Druck auf die Lasche (36a) des ringförmigen Belüftungshebels (36) kann die Mulde wieder entfernt werden, sobald der angehobene Rand der Kappe (38) die Belüftung durch die Rille (35a) freigibt. Meistens genügt hierfür eine Verschiebung des Kappenrandes, der die Rille überdeckt, um nur ca. 2 mm. In dem Ausführungsbeispiel von Figur 3a befindet sich die Lasche (36a) des Belüftungsringes (36) unmittelbar in nächster Lage zu der auslaufenden Belüftungsrille (35a). Es ist aber genauso gut möglich, dass die Lasche (36a) und die Rille (35a) um 180° zueinander versetzt sind. In ersterem Fall muss man zur Belüftung die Lasche (36a) von unten nach oben bewegen und im zweiten Fall in entgegengesetzter Richtung. Beide Möglichkeiten sind äquivalent.

Die Kappe in diesem Ausführungsbeispiel hat einen Innendurchmesser von 26 mm, eine homogene Wandstärke von 1 - 2 mm, sowohl im zylindrischen Teil als auch im leicht konvexen Frontbereich, wo sie allerdings auch etwas dicker oder dünner sein kann, z. B. 2 oder 2,5 mm oder unter 1 mm. Die Länge der Kappe (38) beträgt ca. 23 mm. Die Shore-A Härte des hier verwendeten und bevorzugten additionsvernetzten hochtransparenten Silikonkautschuks liegt vorzugsweise im Bereich 20 - 90, in diesem Beispiel bei 45 (ISO 868). Die relative elastische Dehnbarkeit bis zum Zerreißen: bei ca. 100% (ISO 37).

Material und Geometrie der Kappe sind so gewählt, dass sich deren Andruckfläche bei Andruck auf eine ebene harte Testfläche (mit einem druck von mindestens 0,5 N/cm² um mindestens 5 %, vorzugsweise ca. 10% vergrößert.

Die Kappe wird hergesteilt, indem man die flüssigen Komponenten A und B hier im Verhältnis 9:1 vermischt, dann in der Vakuumkammer blasenfrei macht und alsdann in eine entsprechende Form gießt, in welcher bei erhöhter Temperatur die Vernetzung stattfindet. Man kann die Kappe (38) sehr gut auf den metallenen Tubus (35) aufschieben und wieder entfernen, wenn man den Tubus (35) außen mit einer dünnen Schicht eines hochviskosen Öls (Silikonöl) oder Fetts (Vaseline) versieht, welche man nur einmal oder selten aufbringen muss.

Das Material, aus dem die Kappe (38) besteht, ist höchst transparent, so dass im Bereich von 350 - 980 nm bei einer Schichtdicke von 1 - 2 mm ca. 90% optische Transmission gemessen werden kann. Das ist wichtig, damit keine Strahlungsenergie "verschenkt" wird.

Darüber hinaus ist das Material gewebeverträglich, kann mit allen in der Medizin üblichen Mitteln desinfiziert werden, und kann außerdem auch autoklaviert werden. Das hier verwendete Material kann sogar die medizinische Zulassung nach ISO 10993-1 und nach USP Class VI erhalten. Seine große chemische Inertheit ist ebenfalls von Wichtigkeit weil es in der hier vorliegenden Erfindung mit vielen verschiedenen flüssigen oder cremigen oder pastösen Trägersubstanzen und Wirkstoffen in Berührung kommt.

Man kann die Andruckkappe (18, 28, 38) aus Silikon (oder einem anderen transparentem Material) auch insgesamt mit einem lichtabsorbierenden Färbematerial einfärben. Im Extremfall enthält das Färbematerial feinverteiltes Graphitpulver und die Silikonkappe wird total schwarz. Hierfür genügt eine Beimischung des graphithaltigen schwarzen Färbematerials von nur einem Gewichtsprozent. Eine solche schwarze Silikonkappe wird durch die im Inneren auftreffende LED-Strahlung schnell erwärmt und kann aufgrund der Kontaktwärme die sich auf das Gewebe überträgt ebenfalls zum Einarbeiten von wirkstoffhaltigen Salben (Gelen) verwendet werden. Eine Blendwirkung ist naturgemäß nicht vorhanden.

Das Wirkprinzip der schwarzen Kappe ist aber im Gegensatz zur Aufheizung des Gewebes durch im Gewebe absorbierte Strahlung bei Verwendung einer transparenten Kappe ein völlig anderes: Die schwarze, aufgeheizte Kappe, gibt ihre Wärme durch den langsamen Prozess der Wärmeleitung an das Gewebe ab, im Gegensatz zur momentanen Aufheizung durch im Gewebe absorbierte Strahlung, die auch sofort innerhalb der Strahleindringtiefe wirkt. Ein Vorteil der schwarzen Kappe ist jedoch, dass sie auf der Oberfläche der Kappe aufgetragene wirkstoffhaltige Substanz (213) schnell erwärmt mit der Folge, dass sich deren Viskosität erniedrigt, wodurch sie schneller und tiefer in das Gewebe eindringen kann. Die meisten Salben und Gele werden durch direkte Strahlungsabsorption nicht oder nur langsam aufgeheizt, weil sie im sichtbaren und nahinfraroten Spektralbereich (360 ≤ λ ≤ 950 nm) kaum oder nur wenig absorbieren.

In diesem Zusammenhang ist auch eine graue Kappe interessant, die mit dem graphithaltigen Färbemittel so eingefärbt wird, dass die frontale Lichtaustrittsfläche einen gewissen Anteil des auf sie auftreffenden LED Lichtes noch durchlässt, z. B. 10% - 90%, vorzugsweise 20% - 80%, und der andere Teil der Strahlung in der Lichtaustrittsfläche (18a) der Kappe (18) absorbiert wird und diese erwärmt. Somit hat man die Kombination der beiden Mechanismen der Einschleusung von wirkstoffhaltigen Substanzen im Gewebe zur Verfügung, je nach Wahl der optischen Transmission der Lichtaustrittsfläche der Kappe (18), die auch schnell gegen eine andere mit anderer optischer Transmission ausgetauscht werden kann.

Die Blendwirkung der "grauen" Kappe ist zumindest stark reduziert, insbesondere bei Transmissionswerten von T ≤ 60%, was ebenfalls von Vorteil ist. Die Blendwirkung der "grauen" Kappe kann auch reduziert werden, wenn die Wandstärke der Kappe im zylindrischen Teil größer ist als im Frontbereich.

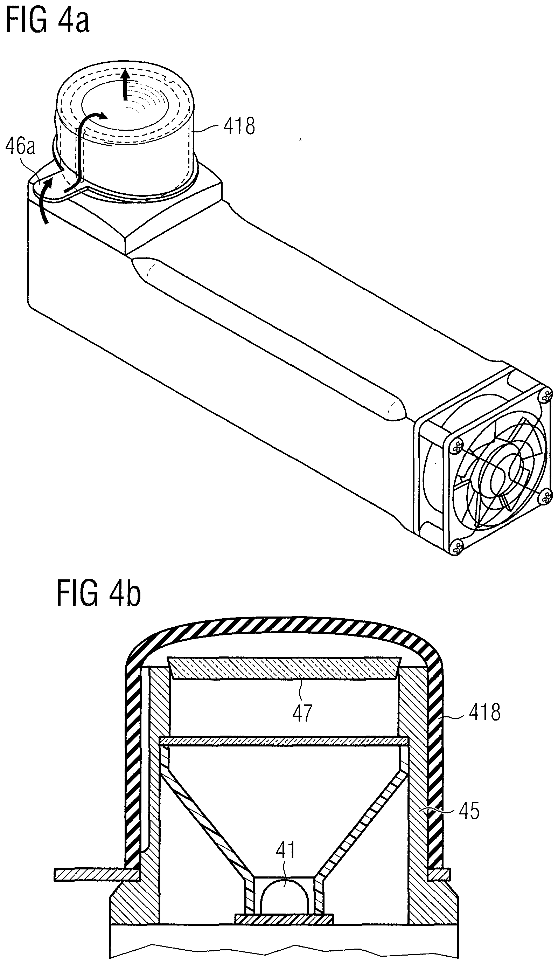

Figur 4a zeigt das dermatologische Bestrahlungsgerät fertig montiert mit aufgesetzter Kappe (418) und leicht angehobener Belüftungslasche (46a) wodurch die Luft von außen durch die Gelüftungsrille in den Innenraum des Tubus gelangen kann und dabei die konkave Form der Lichtaustrittsfläche der Kappe (418) in die konvexe (oder plane) zurückführt.

Figur 4b zeigt die Anbringung der Fluoreszenzplatte (47) im Innenraum des Tubus (45). Die Fluoreszenzplatte, vorzugsweise eine mit einem der Perylenfarbstoffe Lumogen® rot oder Lumogen® gelb oder Lumogen® orange dotierte Plexiglasscheibe, hat innerhalb des Tubus (45) einen lockeren Sitz, weil sie leicht auswechselbar sein soll. Die Silikonkautschukkappe (418) umhüllt hier den Tubus (45) einschließlich des Fluoreszenzplättchens (47) .

Wählt man ein Dioden-Array (41) mit 4 Einzeldioden die alle einheitlich im blauen Spektralbereich zwischen 455 nm und 475 nm emittieren, so wird diese Strahlung vollständig in dem 3 mm dicken Plättchen (47), welches mit Lumogen® rot und/oder gelb oder orange hoch dotiert ist, absorbiert und in längerwellige Fluoreszenzstrahlung umgewandelt die etwa bei 580 nm oder 630 nm ihr spektrales Maximum hat und von den Farbstoffmolekülen in den gesamten Raumwinkel 4 π emittiert wird. Dabei wird die eine Hälfte der Fluoreszenzstrahlung in Vorwärtsrichtung und die andere Hälfte in Rückwärtsrichtung, zurück zur Diode (41) emittiert.

Man kann aber die in die Rückwärtsrichtung gestreute Fluoreszenzstrahlung wieder nach vorne umlenken, indem man vor das Plättchen (47), also diodenseitig, sandwichartig ein Glasplättchen von ca. 1 mm Dicke und gleichem Durchmesser wie das Plättchen (47) vorschaltet, welches mit einem Kurzpass-Dünnschichtfilter bedampft wurde. Dieses Filter hat die Eigenschaft dass die Pump- bzw. Anregungsstrahlung im Bereich 455 nm < λ < 475 nm mit fast 100% transmittiert wird, und die Fluoreszenzstrahlung im Bereich 550 nm < λ < 730 nm zu fast 100% reflektiert wird. Die Kantenlänge des Kurzpassfilters liegt im Bereich 480 nm < λ < 510 nm.

Auf diese Weise kann der nach hinten, in Richtung der Diode (41), emittierte Anteil der Fluoreszenzstrahlung nach vorne in Richtung der Strahlaustrittsöffnung umgelenkt werden, wobei man eine Intensitätssteigerung der nutzbaren Fluoreszenzstrahlung von immerhin 35% messen kann. Eine Steigerung um 100% ist leider, wegen der Eigenabsorption der Fluoreszenzstrahlung in dem mit dem Perylenfarbstoff hoch dotierten Material, nicht möglich.

Bei einer Pumpleistung im Blauen von ca. 2,5 Watt kann man so mit Hilfe der hier beschriebenen Fluoreszenztechnik aus einer Lichtaustrittsöffnung von ca. 25 mm Ø eine Strahlausgangsleistung im gelb-roten Spektralbereich von etwa 700 mW erzielen. Diese Strahlleistungsdichte von etwa 140 mW/cm² reicht schon aus um auf der Haut eine spürbare Wärmeentwicklung zu erzeugen. Die Strahlung im längerwelligen gelben und roten Spektralbereich dringt wesentlich tiefer in das Gewebe ein als die Strahlung im Blaubereich und kann deshalb medizinisch bzw. kosmetisch genutzt werden.

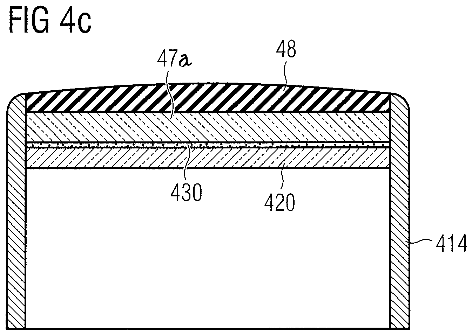

In Figur 4c wird eine zu Figur 4b alternative Anordnung des Fluoreszenzplättchens (47) gezeigt, die es erlaubt mit einfachen Mitteln und schnell von Blauzu Gelb-Rot- oder Rot- oder Weißlicht-Bestrahlung zu wechseln. Das Fluoreszenzplättchen (47a) befindet sich in Figur 4c in einer Aufsteckhülse (414) aus Metall oder Kunststoff, die man auf den Tubus (45) von Figur 4b statt der Kappe (418) aufsteckt. Die Strahlaustrittsfläche des Fluoreszenzplättchens (47a) kann, wie in Figur 4c gezeigt, mit einer dünnen hochtransparenten oder zumindest transluzenten Schicht (48) aus Kunststoff, vorzugsweise wieder aus Silikonkautschuk, überzogen sein.

An die gegenüberliegende Fläche des Fluoreszenzplättchens (47a) kann sich deckungsgleich mit dem Plättchen (47a) ein Dünnschichtfilter (420) anschließen, welches die bereits beschriebene Kurzpasseigenschaft hat, d. h. größtmögliche Transmission im Blaubereich und größtmögliche Reflexion im Gelb-Rot-Bereich des Spektrums. Das Kurzpassfilter (420) kann auf ein Glasplättchen von ca. 1 mm Dicke aufgedampft sein, welches über eine dünne Schicht (430) eines optischen Klebers, z. B. eines Acrylatklebers, mit dem Fuoreszensplättchen (47a) wie in einer Sandwichstruktur verbunden sein kann. Das Sandwich, bestehend aus den beiden Plättchen (420 und 47a) ist im Randbereich mit der ringförmigen Aufsteckhülse (414) verklebt, die ihrerseits mit Klemmsitz auf den Tubus (45) Figur 4b aufgesteckt werden kann.

Das Kurzpassfilter in Dünnschichttechnik kann auch direkt auf das mit Perylen dotierte Plexiglasplättchen (47a) aufgebracht sein, z. B. mit Hilfe der Sol-Gel-Technik. Die Kurzpassfilter mit optimalen optischen Eigenschaften werden allerdings auf Glassubstrate aufgedampft.

Es kann sinnvoll sein, dass das Kurzpassfilter (420) nicht fest mit dem Wellenlängenschieber, sei es in Form eines Fluoreszenzplättchens (17) oder in Form einer mit Fluoreszenzfarbstoffen dotierten Kappe (18), verbunden ist. In diesem Fall kann das Kurzpassfilter auch bei reiner Blaulichtbestrahlung von Nutzen sein, weil es störende längerwellige Untergrundstrahlung der LED abschneidet und deshalb eine kontrastreicherer Fluoreszenzdiagnose (z. B. bei Nagelpilz) mit Hilfe eines Langpassfilters ermöglicht. Dieses ist auch von Vorteil, wenn das Bestrahlungsgerät als forensische Lampe bei der Spurensuche von Blut, Sperma oder Speichel verwendet wird.

Die hier beschriebene Fluoreszenztechnik erlaubt es auf einfache und kostensparende Weise eine einzige LED-Lichtquelle in vier verschiedenen Farbbereichen (Blau, Gelb, Rot, Weiß) mit verschiedener Eindringtiefe im Gewebe für medizinische oder kosmetische Anwendungen oder nur für Beleuchtung, zu verwenden. Die Alternative wäre sonst die Verwendung von vier verschiedenen LED-Strahlungsquellen, was natürlich auch möglich aber kostspieliger ist. Die Fluoreszenztechnik ist nicht beschränkt auf die Verwendung von blauem Licht als Pumpstrahlung. Sie funktioniert hier allerdings besonders gut wegen des maximalen Wirkungsgrades der Dioden im Blaubereich und der Tatsache dass die besonders effizienten Perylen-Fluoreszenzfarbstoffe Lumogen® rot und Lumogen® gelb (oder Lumogen® orange) im Blauen ihre stärkste Absorption bzw. die wirkungsvollste Pumpbande haben.

Man kann die Dicke der Fluoreszenzplättchen (47, 47a) so einstellen, dass die blaue Pumpstrahlung vollständig in längerwellige Fluoreszenzstrahlung umgewandelt wird. Ein solches Plättchen hat dann z. B. eine Dicke von 3 mm. Wählt man eine geringere Plättchendicke von z. B. nur 2 mm oder 1 mm, so geht immer mehr blaue Pumpstrahlung durch das Plättchen (47, 47a) durch, so dass man Mischlicht erhält, welches aus lang- und kurzwelligen Anteilen besteht. Auch eine derartige Mischstrahlung kann dermatologisch oder kosmetisch für Faltenglättung oder andere Anwendungen (z. B. Akne) von Nutzen sein, weil es in der Haut ein weniger scharfes Termperaturprofil erzeugt, d.h. eine etwas geringere Oberflächentemperatur aber dafür eine größere Eindringtiefe bis in die Subkutis.

So hat es sich beispielsweise bewährt, bei der Faltenglättung mit Hilfe Hyaluron®-haltiger Substanzen (Gel, Flüssigkeit) sowohl mit Blaulicht (455 nm ≤ A ≤ 475 nm) als auch mit Rotlicht oder Gelb-Rot-Licht im Bereich 560 nm ≤ A ≤ 780 nm zu bestrahlen. Es ist auch möglich fluoreszierende Farbstoffe direkt in die Silikonkautschukkappe (18, 28, 38, 418) welche mit dem Gewebe kontaktiert einzubringen, um das LED Licht in längerwelliges Fluoreszenslicht umzuwandeln oder zumindest eine längerwellige Farbbeimischung zu dem beispielsweise blauen LED Licht zu erhalten mit dem Ziel einer größeren Eindringtiefe der Strahlung in das Gewebe.

Hierfür eignen sich die Lumogen® Farbstoffe aus der Gruppe der Perylene die sich in den nicht polaren Slikonölen gut lösen und deshalb auch gut in die Silikonkautschukkappe mit eingebaut werden können. Aber auch andere Fluoreszensfarbstoffe lassen sich vor dem Gießen und Vernetzen der Kappe in der Flüssigphase des Silikons mit einbauen.

So ist es möglich zur Erzeugung von längerwelligem Fluoreszenzlicht im gelben und roten Spektralbereich bei Anregung mit Blaulicht in das Material der Gewebeandruckkappe aus Silikon, welches aber auch aus einem anderen transparenten Polymer oder Elastomer oder kautschukartigen Material bestehen kann, außer Lumogen® Farbstoffen auch folgende fluoreszierende Farb- oder Leuchtstoffe einzubauen: Leuchtstoffe auf Basis von seltenen Erden, wie z. B. Cer, Samarium, Europium, Terbium, Neodym und andere, welche ihrerseits meist in einer gläsernen Matrix wie z. B. (Sr, Ba, Ca)₂ SiO₂ oder in kristalliner Matrix, wie in Yttrium-Aluminium-Granat (Y₂Al₅O₁₂) eingebaut, und in fein pulverisierter Form erhältlich sind.

Aber auch Farb- bzw. Leuchtstoffe auf Basis von Übergangsmetallen wie z.B. Ti, Cr, Mn, Fe, Co, Ni Cu usw. lassen sich, wenn sie in einer kristallinen Matrix eingebaut sind und in pulverisierter Form vorliegen in die Gewebeandruckkappe einbauen oder vor der endgültigen Vernetzung (Silikon) bzw. Aushärtung einmischen.

Als Beispiel sei hier ein Leuchtstoff bestehend aus pulverisiertem Rubin, d.h. Chromionen in einer Matrix aus kristallinem Al₂O₃ genannt. Dieser Leuchtstoff absorbiert im blauen und violettem Spektralbereich und fluoresziert im langwelligen roten Spektralbereich bei 694 nm.

Oder man inkorporiert in das Silikon der Gewebeandruckkappe fein pulverisiertes Plexiglas (Acrylglas) oder andere pulverisierte transparente Kunststoffe welche mit einem Perylen Farbstoff (Lumogen®) dotiert wurden, so dass sich die Perylen-Moleküle in einer Matrix aus Acrylglas oder einem anderen Kunststoff befinden, was von Vorteil ist, weil die Perylene in einer solchen Matrix wie Acrylglas besonders photostabil sind und besonders effektiv fluoreszieren.

Des weiteren können in die Andruckkappe auch fluorezierende Leuchtstoffe auf der Basis von Quantum Dots, wie z. B. CaTe, oder (Cd, Se) ZnS oder Pb Se, auch als Nanopigmente, eingebaut werden.

Man kann die mit Leuchtstoffen dotierten Gewebeandruckkappen aus Silikon nach der Vernetzung noch zusätzlich, vor allem auf der Gewebeandruckfläche mit einer dünnen Schicht aus Silikon beschichten (Dicke der Schicht ≈ 0,1 mm - 1 mm), um zu vermeiden, dass die Leuchtstoffe mit dem Gewebe in Berührung kommen.

Zu allen diesen Farb- bzw. Leuchtstoffen in Silikon® oder anderen transparenten Polymeren oder Elastomeren, kann man noch feingekörntes SiO₂-Pulver beimischen um die Homogenität und die Effektivität der emittierten Fluoreszensstrahlung zu verbessern. Das SiO₂-Pulver kann hier bis zur feinst möglichen Körnung im Nanobereich verwendet werden.

So kann man mit einer Silikonkappe welche z. B. mit den seltenen Erdfarbstoffen Eu und/oder Ce dotiert ist, noch mit einem Zusatz von SiO₂-Pulver, bei Anregung mit einer LED, welche im Blaubereich bei ca. 460 nm emittiert, sehr homogenes diffuses Weißlicht erzeugen, welches sich auch als Diagnoselicht hervorragend eignet, und dies nicht nur bei dermatologischen Anwendungen sondern auch z. B. in der Forensik.

SiO₂-Pulver allein in der Silikonkappe, ohne Zusatz eines weiteren Leuchtstoffes wirkt als Lichtdiffusor und kann in speziellen Fällen bei der Applikation der Lichtstrahlung z. B. in Körperhöhlen nützlich sein.

Der Vollständigkeit halber soll erwähnt werden, dass man auf die Kappendeformation, wie anhand der Fig. 2 - 3 beschrieben, auch verzichten kann, insbesondere bei Verwendung von weniger hochwertigen und teueren Gelen (Salben, Pasten), oder bei Verwendung von Kappen mit größerem Durchmesser, weil diese mit Gelen (Pasten, Salben) leichter beladen werden können. Solche größeren Kappen haben z.B. einen Durchmesser der Gewebeandruckfläche von > 10 mm, beispielsweise 40 mm.

Die Andruckkappen sind vorzugsweise fest mit einem ringförmigen Unterbau oder Sockelelement aus Kunststoff (z. B. einem Duroplast wie Delrin®) oder Metall verbunden, die ihrerseits auf den Tubus (15) von Fig. 1 mit Klemmsitz aufgesteckt werden können, so dass die verschiedenen Kappen verschiedener Größe und mit unterschiedlichen spektralen Eigenschaften schnell gegeneinander ausgetauscht werden können, Siehe dazu auch die Figuren 6d und 6e. Auch Lichtleitstäbe einschließlich der optisch isolierenden Umhüllung, gemäß der Figur 6a können auf so einem Sockelelement (6d24) aufmontiert sein, so dass ein schneller Wechsel von Kappen mit geringer Strahlleistungsdichte zu kleinflächigen Applikationen mit hoher Strahlleistungsdichte möglich ist.

Blaulicht dringt zwar lediglich in die Dermis ein, wogegen längerwelliges Licht ab λ > 500 nm auch bis in die Subkutis eindringt. Allerdings steht bei Blaulicht emittierenden Lumineszenzdioden eine ca. 3x höhere Strahlungsleistung zur Verfügung, bei gleicher elektrischer Leistung (z. B. 10 - 15 Watt) wegen des maximalen optischen Wirkungsgrades der LEDs im Blauen. Aus diesem Grund kann man den Einschleusungseffekt des Wirkstoffes in die obersten Hautschichten am schnellsten mit intensivem Blaulicht im Leistungsdichtebereich von ca. 100 - 1000 mW/cm² erreichen.

Die ständig gleitende Bewegung der Strahlungsquelle auf der Hautoberfläche ist bei dieser hohen Strahlleistungsdichte erforderlich, weil das Gewebe sonst nach ca. 5 - 10 Sekunden statischer Bestrahlung zu heiß würde. Gerade diese sofort spürbare Wärmeentwicklung im intensiven Blaubereich des Spektrums ist es aber, welche die Mikrozirkulation in der obersten Hautschicht (bis zu ca. 1 mm Tiefe) rasch anregt und damit die Einschleusung des Wirkstoffs in das Gewebe beschleunigt bzw. verbessert.

Die geringe Eindringtiefe des blauen Lichtes im Vergleich zum Rotlicht hat auch den Vorteil, dass man z. B. bei der Faltenbehandlung, näher im Bereich des Auges arbeiten kann, ohne dass das Auge durch vagabundierendes Streulicht irritiert wird.

Selbstverständliche Voraussetzung bei der Anwendung solcher intensiven Lichtquellen ist natürlich das Tragen von Schutzbrillen sowohl für den Patienten als auch für den Behandler. Bewährt hat sich auch das Tragen von Solarium-Brillen seitens des Patienten während der Behandlung.

Statt der Fluoreszenztechnik mit Perylenfarbstoffen oder anderen auch anorganischen Farbmolekülen kann man auch das erfindungsgemäße dermatologische Bestrahlungsgerät mit einem Dioden-Array mit Emission im roten oder nahen infraroten Spektralbereich verwenden, z. B. im Bereich 600 nm < λ < 980 nm. So hat sich bei der Faltenbehandlung mit Licht ein Dioden-Array mit ca. 10 Watt elektrischer Leistung und einer Strahlungsleistung von ca. 1 Watt bei einer Peak-Wellenlänge von 740 nm bewährt.

Diese Strahlung hat den Vorteil, dass sie im Spektralbereich der maximalen Eindringtiefe in Gewebe liegt, aber trotzdem noch sichtbar ist, dabei aber nicht so blendet wie kurzwelligere rote Strahlung (z. B. bei 630 nm), die auch weniger tief eindringt. Noch längerwelligere Strahlung im nahen IR-Bereich dringt auch nicht tiefer ins Gewebe ein (als 740 nm), ist aber unsichtbar und damit gefährlich, vor allem, bei Anwendungen im Gesichtsbereich. Man kann ohne weiteres leistungsstarke Dioden-Arrays mit Emission bei z. B. 850 nm oder 940 nm verwenden in Bereichen außerhalb des Gesichtes. Schutzbrillen sind aber in jedem Falle erforderlich.

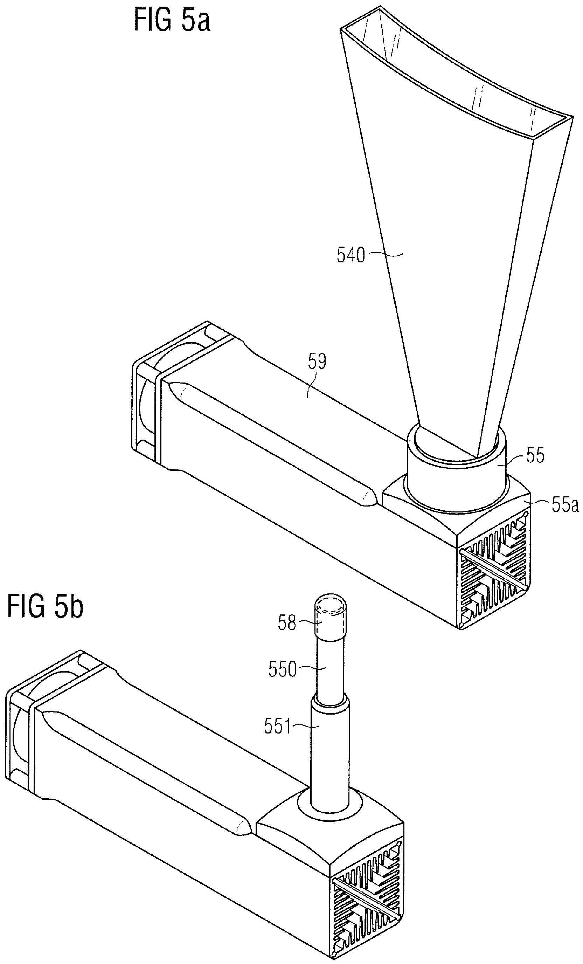

Figuren 5a und 5b zeigen Varianten des erfindungsgemäßen Bestrahlungsgerätes welche die Basiseinheit bestehend aus dem Handgriff (59) mit Lüfter und Lumineszenzdioden-Array, im Prinzip wie in Figur 1 dargestellt, verwenden. Sie unterscheiden sich lediglich durch besondere Applikatoraufsätze (540 bzw. 551) welche spezielle Strahlumformungen der von dem Dioden-Array emittierten Strahlung erlauben.

Figur 5a enthält als Applikatoraufsatz einen Querschnittswandler, welcher aus der kreissymmetrischen Emissionsstrahlung des Dioden-Arrays ein länglich rechteckiges Strahlprofil herstellt, so wie es in der Zahnheilkunde zum Bleachen der Zähne im "Smile Bereich" verwendet werden kann.

Der Strahlumformer (540) auch Strahlquerschnittswandler genannt, besteht hier aus einem pyramidenstumpfförmigen innen hochglanzverspiegeltem Hohlkörper, welcher in den Tubus (55) mit Sockel (55a) eingepasst ist. Die kleinere Basisfläche des Pyramidenstumpfes, welche offen ist, bildet das Strahleintrittsfenster für die Diodenstrahlung, während die größere Basisfläche, welche das Strahlaustrittsfenster bildet, leicht gekrümmt ist und mit einem dünnwandigen transparenten Austrittsfenster abgedeckt ist (hier nicht dargestellt), so dass man den Querschnittswandler bis auf wenige Zentimeter Abstand zum Front- und Seitenzahnbereich ("Smile Bereich") des Patienten einjustieren kann. Das rechteckig streifenförmige Profil der vom Querschnittswandler emittierten Strahlung überdeckt dabei den "Smile Bereich" des Patienten.

Verwendet man als Strahlungsquelle ein einziges Dioden-Array bestehend aus 4 Einzeldioden mit Peakemission bei ca. 395 nm und einer elektrischen Eingangsleistung von ca. 15 Watt, so hat man für das Bleachen auf der Zahnoberfläche eine Strahlleistungsdichte von ca. 100 mW/cm² zur Verfügung, was für die effiziente Radikalbildung in den üblichen peroxydhaltigen Pasten, welche für das Bleachen verwendet werden, vollkommen ausreicht.

Man kann für denselben Zweck auch Dioden-Arrays mit Emission bei 385 nm, oder 360 nm aber auch bei 455 - 470 nm verwenden. Den Querschnittswandler, welcher hier für das Bleachen verwendet wird, kann man sehr einfach herstellen, so wie ich es in meiner früheren Patentanmeldung DE 10 2010 050 962.0 beschrieben habe, so dass die gesamte Bleaching-Lampe von Figur 5a insgesamt sehr günstig herzustellen ist und darüber hinaus auch räumlich klein und leicht gehalten werden kann. Die Offenbarung der Herstellung und Verwendung des Querschnittswandlers in der Patentanmeldung DE 10 2010 050 962.0, insbesondere bezüglich deren Figuren 12 bis 14, wird hiermit durch Bezugnahme vollumfänglich in die vorliegende Anmeldung aufgenommen.

Figur 5b und die Figuren 6a und 6b zeigen die Ankoppelung eines starren oder flexiblen Lichtleiters (550) an die LED bzw. das LED-Array, welche(s) sich in thermischem Kontakt auf dem Handstück mit Kühlung befindet. Der starre oder flexible Lichtleiter (550) wird durch einen rohrförmigen Ansatz mit Sockel (551) gehaltert. Die Lichteintrittsfläche des Lichtleiters (550) bzw. (650) befindet sich praktisch in Berührung mit der Glaskuppel bzw. dem Dom (61) des LED-Arrays.

Der Lichtleiter ist in diesem Beispiel ein Quarzglasstab welcher eine polierte Zylinderfläche und ebenfalls polierte Endflächen aufweist. Der Lichtleitstab (652) der z. B. aus Quarzglas oder Plexiglas bestehen kann wird optisch durch einen eng anliegenden Teflon® FEP- oder Teflon® MFA-Schlauch (653) isoliert, der auf seiner Innenoberfläche eine Teflon® AF (Amorphous Fluoropolymer)-Beschichtung aufweisen kann.

Zusätzlich kann sich zwischen der Innenwandung des Isolierschlauches (653) und der Zylinderoberfläche des Lichtleitstabes (652) noch eine dünne Schicht einer hochviskosen perfluorierten Flüssigkeit, beispielsweise eines Perfluorpolyäthers befinden, der infolge seines extrem niedrigen Brechungsindex im Bereich von n ≈ 1,28 - 1,32 als optische Immersionsflüssigkeit zu der festen Teflon® AF-Schicht wirkt, die ebenfalls einen extrem niedrigen Brechungsindex in diesem Wertebereich hat. Damit ist für den Lichtleitstab (652) eine sehr hohe optische Apertur gegeben (2 α ∼ 83° im Falle von Quarzglas, und 2 α ∼ 93° im Falle von Plexiglas) wodurch ein besonders hoher Raumwinkelanteil an Strahlung des hochdivergent emittierenden Dioden-Arrays von dem Lichtleiter aufgefangen und nach außen geleitet werden kann.

Diese beschriebene Art der optischen Isolation eines Lichtleitstabes aus Glas oder Kunststoff erlaubt im Falle von Plexiglas oder einem anderen transparenten Kunststoff als Material für den Lichtleitstab sogar noch eine gewisse Flexibilität des Lichtleitstabes (652) ohne Gefahr vor Splittern oder scharfen Kanten im Falle von Bruch.

So kann man einen Lichtleitstab (652), welcher z. B. aus Plexiglas besteht und eine Länge von 1000 mm und einen Durchmesser von bis zu ca. 6 mm hat, der optisch mit einem dünnwandigen (d = 0,5 mm) Teflon® FEP-Schlauch ummantelt ist, der auf seiner Innenoberfläche eine Teflon® AF- oder Hyflon® AD-haltige Schicht aufweist, welche außerdem noch eine hochviskose perfluorierte Flüssigkeit enthalten kann, ohne nennenswerten Verlust an Transmission um bis zu 90° biegen. Sollte so ein längerer Lichtleitstab brechen, so schützt der ihn umgebende anliegende Mantelschlauch aus perfluoriertem Kunststoff, wie z. B. aus Teflon® FEP oder Teflon® MFA, der z. B. eine Wandstärke von 0,3 - 1,0 mm haben kann, vor Splittern und Verletzung durch scharfe Bruchkanten. Auch für den Fall dass der Lichtleitstab aus Glas oder Quarzglas besteht, ist für Stabdurchmesser bis zu 2 mm die Biegsamkeit gegeben.

Der optisch und mechanisch isolierende Isolationschlauch aus Teflon® FEP oder MFA (653) des starren bzw. semiflexiblen oder flexiblen Lichtleiters (652) kann aber muss nicht unbedingt eine feste amorphe perfluorierte Innenschicht aufweisen, die z.B. aus Teflon® AF ist. Es genügt auch, dass sich zwischen der Umfangsfläche des Lichtleiterstabs (652) und der Innenoberfläche des Isolations schlauches (653) lediglich eine dünne Schicht der perfluorierten hochviskosen Flüssigkeit befindet.

Diese Flüssigkeit kann z. B. ein perfluorierter hochviskoser Polyäther sein, welcher einen Siedepunkt von über 200°C hat und einen äußerst niedrigen Brechwert im Bereich von ca. 1,28 - 1,32. Derartige Flüssigkeiten sind unter dem Handelsnamen Krytox® (Dupont) oder Fomblin® (Ausimont) im Handel. In diesem Falle wirkt die perfluorierte Flüssigkeit als Immersionsmittel zu dem perfluorierten Manteischlauch (z. B. Teflon® FEP oder MFA), der zwar auch einen niedrigen Brechungsindex hat (n = 1,34) aber nicht wie bei Teflon® AF vollkommen transparent ist. Bei Lichtleiterlängen von bis zu 10 - 100 cm bewirkt diese Vereinfachung der optischen Isolation noch keine merkliche Verminderung der Transmission des Lichtleiters.

Figur 6b zeigt noch eine optische Immersionsschicht (654) weiche die emittierende Glaskuppel (Dom) des LED-Arrays mit der Lichteintrittsfläche des Lichtleiters (652) optisch verbindet. Das Volumen zwischen der Lichteintrittsfläche des Lichtleiters (652) und der LED, bzw. des Doms, ist mit dem Material der Immersion (654) vollständig ausgefüllt.

Die Immersionsschicht (654) kann hier z.B. aus einem hochtransparentem weichen elastischen Silikongel oder Silikonkautschuk mit geringer Shore A Härte (Shore A < 100) oder aus Polymethylmetacylat bestehen. Mit Hilfe der Immersionsschicht kann die Lichteinkoppelung verbessert werden, da das Material der optischen Immersionsschicht von dem Lichtleiter-Isolationsschlauch (653) begrenzt wird. Zwischen der Immersionsschicht (z. B. Teflon® AF) des Isolationsschlauches und dem Material der Immersion besteht ein großer Unterschied bzgl. der Höhe des Brechungsindex. Somit kann ein großer Teil der Strahlung der LED durch Lichtleiteffekt in den Lichtleiter (652) gelangen, welche ansonsten verloren wäre.

Die Figuren 5b, 6a und 6b zeigen eine Kappe (58, 68), die auf das Lichtaustrittsende des Lichtleiters (650) aufgesetzt wird. Sie besteht aus dem gleichen Material wie in den Figuren 1 - 4 beschrieben. Sie erlaubt den direkten Gewebekontakt mit der Lichtaustrittsfläche der Lichtleiteranordnung und kann leicht ausgewechselt werden.

In einem praktischen Ausführungsbeispiel wird ein Lichtleiterstab aus Quarzglas von 10 cm Länge und 8 mm Durchmesser, optisch isoliert durch einen enganliegenden mit Teflon® AF beschichteten Teflon® FEP-Schlauch mit einer Immersionszwischenschicht aus Krytox® 16350 an ein Dioden-Array bestehend aus 4 Einzeldioden gekoppelt, welche im Bereich von 460 nm emittieren.

Das LED-Array hat eine elektrische Leistung von 15 Watt, und die gesamte emittierte Strahlung eine Leistung von ca. 3 Watt. Die Strahlausgangsleistung gemessen am Lichtleiterende beträgt immerhin 2,8 Watt, während sich eine Leistungsdichte unmittelbar an der Lichtaustrittsfläche des Lichtleiters von 5,6 Watt/cm² ergibt. Mit dieser Leistungsdichte lassen sich bereits einige wichtige dermatologische Indikationen (Besenreiser, Altersflecken, Warzen etc.) durch die thermische Wirkung der Strahlung bei Gewebeandruck behandeln, eine Anwendung die man ansonsten nur mit dem Laser macht.

Die optische Isolation des Isolationsschlauchs (653) kann bis zu drei verschiedene fluorierte Polymere enthalten. Der Lichtleiter (652) besteht beispielsweise aus einem homogenen allseits polierten Stab aus Quarzglas (652) mit zylindrischer Symmetrie. Auf seiner Mantelfläche befinden sich vorzugsweise eng anliegend drei Isolationsschichten:

Die erste direkt mit der Mantelfläche des Lichtleiters (652) in Kontakt stehende Schicht besteht aus einer Flüssigkeit bzw. aus einem flüssigen Polymer welche(s) perfluoriert ist, hochviskos ist und einen extrem hohen Siedepunkt hat (Tₛ > 200°C). Perfluorpolyäther sind solche Flüssigkeiten.

Als Beispiel für solche Flüssigkeiten sind Krytox®, Fombline® und Galden® genannt. Die Flüssigkeit Krytox® 16350 ist bespielsweise geeignet

An diese Schicht grenzt eine dünne Schicht (d ∼ 1λ - 3λ) eines festen amorphen perfluorierten Polymers. Teflon® AF oder Hyflon® AD oder Perfluoroalkyl-Vinyläther mit erhöhtem Copolymeranteil sind mögliche Materialien.

Die äußere Schicht ist ein Isolationsschlauch dessen Innenoberfläche mit der Schicht aus dem amorphen perfluorierten Polymer beschichtet ist. Der Schutzschlauch besteht vorzugsweise aus einem Kohlenstofffluorpolymer und hat eine Wandstärke von ca. ¹/₁₀ - ¹⁰/₁₀ mm.

Perfluorierte Polymere wie Teflon® FEP, Teflon® MFA, Teflon® PFA, Teflon® PTFE sind besonders bevorzugte Materialien für den Isolationsschlauch. Aber auch teilfluorierte Polymere wie z. B. das Terpolymer Hostaflon® TFB lassen sich als Material für den Isolationsschlauch einsetzen, wegen der besseren Flexibilität dieser Schläuche.

Aus Kostengründen kann man die feste amorphe innenbeschichtung, z. B. die aus Teflon® AF, auch weglassen und nur die Schicht aus dem perfluorierten oder teilfluorierten flüssigen Polymer als direktes Kontaktmedium mit der Mantel-des Lichtleiters (652) verwenden. Diese weniger aufwendige optische Isolation ist zwar auch etwas weniger wirksam, ist aber für Lichtleiterlängen bis zu 20 cm immer noch sehr gut.

Generell sollte die Immersionsschicht mit der nächstfolgenden Isolationsschicht (amorphe Schicht oder Isolationsschlauch) eine zumindest angenäherte Indexanpassung herbeiführen.

Die flüssige Isolationsschicht hat auch einen großen Montagevorteil. Sie wird vor der Verkleidung des Lichtleiters auf die Mantelfläche des Lichtleiters (652) aufgebracht. Der Lichtleiter lässt sich dann leicht in den eng sitzenden Isolationschlauch (653) reinschieben. Die Flüssigkeitsschicht kann wegen ihrer hohen Viskosität und wegen ihres hohen Siedepunktes auf Dauer in der Lichtleiterstruktur verbleiben. Diese optische Isolationstechnik ist nicht nur für starre Lichtleitstäbe aus Quarzglas, Glas oder transparentem Kunststoff sondern auch für flexible Lichtleitfasern aus Quarzglas, Glas oder für schon optisch mit geringem Aperturwinkel isolierte Lichtleitfasern z. B. aus Quarzglas oder für optische Fasern aus Plexiglas® verwendbar.

Um den Dom der LED (61) kann noch ein innen verspiegeltes Röhrchen (nicht gezeigt) angeordnet sein, welches z. B. aus Aluminium hergestellt ist. Das Röhrchen umhüllt sowohl auf einigen Millimetern Länge den Lichteintrittsbereich des Lichtleiters (652) mit möglichst geringem Spalt zwischen dem Innenlumen des Röhrchens und der Umfangsfläche des Lichtleiters, als auch den Dom aus Glas oder Kunststoff der LED (61) so weit wie möglich. Im Idealfall reicht das Röhrchen bis zur Bodenplatte, dem PCB-Board der LED. Die Verspiegelung auf der Innenfläche des Röhrchens kann elektrolytisch, oder durch Aufdampfen, oder durch Beklebung der Innenfläche des Röhrchens mit einer reflektierenden Folie erfolgen. In der eben beschriebenen Anordnung bewirkt das Röhrchen eine um ca. 25% gesteigerte Lichtausgangsfeistung aus dem Lichtleiter (650), die für die effektive dermatologische Behandlung, insbesondere von Akne, Altersflecken, Warzen oder Besenreisern besonders erwünscht ist.

Die Verbindung eines starren Lichtleiters mit dem Dioden-Array in der hier beschriebenen hocheffizienten Weise erlaubt auch die Verwendung der LED-Strahlung in Körperhöhlen (Nase, Ohr, Rachenraum etc.), weil höhere Strahlleistungsdichten näher an den Ort der Behandlung herangeführt werden können. Es ist ebenfalls möglich das Gerät aus Figur 5b, 6a, 6b in der Zahnheilkunde für die Polymerisation von Kunststofffüllungen oder für industrielle Anwendungen zum Aushärten von lichthärtenden Kunststoffen auf Basis von Epoxiden, Acrylaten oder Silikonelastomeren zu benützen. So erlaubt es die weiche, anpassungsfähige Kappe (58), das Lichtleiterende bei der Polymerisation einer Dentalfüllung auf die Füllung aufzusetzen, auch unter Druck, wobei die höchste Strahlleistungsdichte genutzt werden kann und die Sauerstoffinhibierung der Polymerisation an der Oberfläche durch mechanische Verdrängung des Luftsauerstoffes reduziert werden kann.

Man kann die Kappe (58) in Figur 5b aus weichem hochtransparentem Silikonkautschuk auch als Einwegkappe benutzen, so dass die Lichtaustrittsfläche des starren Lichtleiters, der auch ein am Lichtaustrittsende etwas gekrümmter starrer Faserstab sein kann, stets sauber und mit maximaler Durchlässigkeit für die Polymerisationsstrahlung, zur Verfügung steht.

Die Kappe (58) in Figur 5b oder (68) in den Figuren 6a und 6b kann auch die Form eines länglichen Strumpfes haben (auch auswechselbar als Einwegteil), der bei medizinischen Anwendungen, infolge seiner Sterilisierbarkeit oder Autoklavierbarkeit, den Kontakt des starren Lichtleiters (550), (650) mit dem Gewebe erlaubt.

Bei Anwendungen des Bestrahlungsgerätes gemäß den Figuren 1 - 6 für die Licht- oder UV-Härtung, beispielsweise von Materialien auf Epoxid-Acrylat- oder Silikonelastomer Basis bei denen die Strahlaustrittsfläche in Kontakt mit dem zu härtenden Kunststoff kommen kann oder soll, kann es wegen der besseren Antihafteigenschaft auch von Vorteil sein, eine Kappe zu verwenden deren Strahlaustrittsfläche aus einem Fluor-Kohlenstoff-Polymer besteht. Besonders vorteilhaft sind hierfür perfluorierte Polymere wie z. B. Teflon® FEP oder Teflon® MFA. So transmittieren dünne Folien (d = 0,5 mm) aus diesen Materialien sowohl im UVA- als auch im sichtbaren Spektralbereich über 80%, und sind ebenfalls für medizinsche Anwendungen sterüisierbar und autoklavierbar.

## Patentansprüche

1. Gerät zur dermatologischen oder kosmetischen Behandlung eines Patienten, mit einem Basiskörper (19; 6c19), der eine Strahlungsquelle (11 ; 21 ; 41 ; 61 ; 6c1 1) zur Emission von Licht in einem Wellenlängenbereich zwischen 320 nm und 1500 nm aufweist, einem Zwischenstück (15; 25; 35; 45; 550; 551 ; 650; 6c15; 6c25), das auf dem Basiskörper (19; 6c19) aufgesetzt ist, um das Licht von der Strahlungsquelle (11; 21; 41; 61; 6c1 1) in Richtung eines zu behandelnden Körperbereichs (214) des Patienten zu leiten, und einem am Lichtaustrittsende des Zwischenstücks (15; 25; 35; 45; 55; 550, 650; 6c15, 6c25) angeordneten Aufsatzelement (18; 28; 38; 414; 418; 58; 68; 6c18; 6d18; 6e18), das dazu ausgelegt ist, in unmittelbaren Kontakt mit dem zu behandelnden Körperbereich (214) gebracht zu werden, wobei das Aufsatzelement (18; 28; 38; 414; 418; 58; 68; 6c18) klemmend an der äußeren Umfangswand des Lichtaustrittsendes des Zwischenstücks sitzt und wobei das Aufsatzelement bei Andruck gegen den zu behandelnden Körperbereich (214) verformbar ist **dadurch gekennzeichnet, dass** das Aufsatzelement (18; 28; 38; 414; 418; 58; 68; 6c18; 6d 8; 6e 8) in Form einer ein Elastomer oder einen fluorierten thermoplastischen Kunststoff enthaltenden Kappe ausgebildet und über das Lichtaustrittsende des Zwischenstücks (15; 25; 35; 45; 550, 551 ; 650, 6c25) gestülpt ist, und dass das Aufsatzelement (18; 28; 38; 414; 418; 58; 68; 6c18; 6d18; 6e18) wenigstens an seiner Lichtaustrittsfläche vollständig aus dem Elastomer oder dem fluorierten thermoplastischen Kunststoff besteht.

2. Gerät nach Anspruch 1, ferner mit einem Sockelelement (6c24; 6d24; 6e24) mit zylindrischer Grundform, das abnehmbar auf einen Grundkörper des Zwischenstücks (15; 25; 35; 45; 6c15, 6c25) aufgesteckt ist, wobei eine starre oder flexible Lichtleiteranordnung (550; 650) oder das Aufsatzeiement (6c18; 6d 18; 6e18) beispielsweise durch Verkleben fest mit dem Sockelelement (6c24; 6d24; 6e24) verbunden ist.

3. Gerät nach Anpruch 2, wobei das Sockelelement (6e24) einen ersten Abschnitt zum Aufstecken mit Klemmsitz auf den Grundkörper des Zwischenstücks (15; 25; 35; 45; 6c15, 6c25) und einen zweiten Abschnitt (6e24a) zum Befestigen bzw. Aufkleben der Lichtleiteranordnung (550; 650) oder des Aufsatzelements (6e 8) aufweist.

4. Gerät nach einem der vorstehenden Ansprüche, wobei das Elastomer eine Shore-A Härte von weniger als 100, vorzugsweise zwischen 30 und 70, insbesondere etwa 45, hat.

5. Gerät nach einem der vorstehenden Ansprüche wobei wenigstens die Lichtaustrittsfläche des Aufsatzelements (18; 28; 38; 414; 418; 58; 68; 6c18; 6d18, 6e18) aus einem hochtransparenten Material, insbesondere einem additionsvemetzten Silikon-Elastomer besteht.

6. Gerät nach einem der vorstehenden Ansprüche, wobei das Aufsatzelement (18; 28; 38; 414; 418; 58; 68; 6c18; 6d18; 6e18) eine lichtabsorbierende Substanz ohne Fluoreszenzeigenschaft, insbesondere Kohle oder Graphit, enthält.

7. Gerät nach Anspruch 6, wobei das Aufsatzelement (18; 28; 38; 414; 418; 58; 68; 6c18; 6d18; 6e18) 0,01 bis 3 Gew.-% der lichtabsorbierenden Substanz ohne FSuoreszenzeigenschaft enthält.

8. Gerät nach Anspruch 6 oder 7, wobei die lichtabsorbierende Substanz ohne Fluoreszenzeigenschaft zwischen 10% und nahezu 100% des von der Strahlungsquelle (11; 21; 41; 61; 6c11) emittierten Lichts absorbiert und in Wärme umwandelt.

9. Gerät nach einem der vorstehenden Ansprüche, wobei das Elastomer aus einem der folgenden Materialien ausgewählt ist; Synthetikkautschuk, Silikonkautschuk, insbesondere additionsvernetzter Silikonkautschuk, Polyurethan oder Polyethylen.

10. Gerät nach einem der vorstehenden Ansprüche, wobei der fluorierte thermoplastischen Kunststoff aus einem der folgenden Materialien ausgewählt ist: Fluor-Kohlenstoff Polymere wie Teflon<®> FEP, Teflon® MFA, Teflon<®> PTFE, Hyflon<®> THV.

11. Gerät nach einem der vorstehenden Ansprüche, wobei das Aufsatzelement (18; 28; 38; 414; 418; 58; 68; 6c18; 6d18; 6e18) die Form einer Kappe hat, deren Gewebeandruckfläche (18a) zwischen einer konvexen und einer konkaven Konfiguration variierbar ist.

12. Gerät nach einem der vorstehenden Ansprüche, wobei das Aufsatzelement (18; 28; 38; 414; 418; 58; 68; 6c18; 6d18; 6e18) eine lichtabsorbierende Substanz mit Fluoreszenzeigenschaft, insbesondere Leuchtstoffe auf Basis von PerylenLeuchtstoffen, insbesondere Lumogen®, oder seltene Erden, enthält.

13. Gerät nach Anspruch 12, wobei das mit der lichtabsorbierenden Substanz dotierte Aufsatzelement (18, 28, 38, 414, 418, 58, 68; 6c18, 6d18, 6e18) wenig stens auf seiner Gewebeandruckfläche (18a) mit einer dünnen Schicht aus undotiertem Silikon beschichtet ist.

14. Gerät nach einem der vorstehenden Ansprüche, ferner mit einem Mechanismus (6c20, 6c21), der dazu ausgelegt ist, die Spannungsversorgung der Strahlungsquelle (6c1 1) zu unterbrechen oder zu vermindern, wenn die Andruckkraft des Aufsatzelements (6c18; 6d18; 6e 8) gegen den zu behandelnden Körperbereich (214) unterhalb eines vorbestimmten Schwellenwerts liegt, und die Spannungsversorgung der Strahlungsquelie (6c11) herzustellen oder zu erhöhen, wenn die Andruckkraft des Aufsatzelements (6c18; 6d18; 6e18) gegen den zu behandelnden Körperbereich (214) oberhalb des vorbestimmten Schwellenwerts liegt.

15. Gerät nach Anspruch 14, wobei ein Grundkörper des Zwischenstücks (6c15, 6c22, 6c23, 6c25) einen fest an dem Basiskörper (6c19) befestigten Verbindungskörper (6c15) und einen relativ zu dem festen Verbindungskörper (6c15) bewegbar gelagerten Verbindungskörper (6c25) aufweist.

16. Gerät nach Anspruch 15, wobei der Mechanismus (6c20, 6c21) ein Schaltelement (6c20) aufweist, das zwischen dem bewegbaren Verbindungskörper (6c25) und dem Basiskörper (6c19) angeordnet und dazu ausgelegt ist, bei einer Andruckkraft des Aufsatzelements (6c18; 6d18; 6e18) gegen den zu behandelnden Körperbereich (214) über dem vorbestimmten Schwellenwert, von dem bewegbaren Verbindungskörper (6c25) in einen Zustand geschaltet zu werden, durch den die Strahlungsquelle (6c1 1) an die Spannungsversorgung angeschlossen oder die Spannungsversorgung der Strahlungsquelle (6c1 1) erhöht wird.

17. Gerät nach Anspruch 16, wobei der Mechanismus (6c20, 6c21) ferner ein elastisches Element (6c21) aufweist, das zwischen dem bewegbaren Verbindungskörper (6c25) und dem Basiskörper (6c19) angeordnet und dazu ausgelegt ist, den bewegbaren Verbindungskörper (6c25) von dem Basiskörper (6c 9) weg zu drücken und dadurch das Schaltelement (6c20) in einen Zustand zu schalten, in dem es die elektrische Verbindung der Strahlungsquelle (6c1 1) mit der Spannungsquelle unterbricht, wenn die Andruckkraft des Aufsatzelements (6c18; 6d18; 6e18) gegen den zu behandelnden Körperbereich (214) unter dem vorbestimmten Schwellenwert liegt, und das elastische Element (6c21) insbesondere eine Feder ist, bspw. eine Spiralfeder.

18. Gerät nach einem der vorstehenden Ansprüche, wobei die Strahlungsquelie (1 1; 21 ; 41 ; 61 ; 6c1 1) eine LED oder ein LED-Array aufweist, deren elektrische Gesamtleistung vorzugsweise zwischen 5 und 25 Watt beträgt.

19. Gerät nach einem der vorstehenden Ansprüche, wobei die Strahlungsquelle (1 1; 21 ; 41 ; 61; 6c1 1) Licht im Wellenlängenbereich 320 nm < λ < 1500 nm und vorzugsweise zwischen 350 nm und 485 nm emittiert.

20. Gerät nach einem der vorstehenden Ansprüche, das dazu ausgelegt ist, eine dermatologisch oder kosmetisch wirksame Substanz (213) unter Bestrahlung mit dem emittierten Licht in den zu behandelnden Körperbereich (214) einzubringen.

21. System mit einem Gerät nach einem der vorstehenden Ansprüche und mindestens einem weiteren Aufsatzelement, das sich zum Austausch des Aufsatzelements (18; 28; 38; 414; 418; 58; 68; 6c18; 6d18; 6e18) eignet und sich von diesem nur hinsichtlich der Größe und der Lichtransmissionseingenschaften unterscheidet, und/oder mindestens einer Lichtleiteranordnung (550; 650) zum austauschbaren Aufstecken auf einen Grundkörper des Zwischenstücks (15; 25; 35; 45; 6c 5, 6c25).

22. System nach Anspruch 21, wobei die Aufsatzelemente unterschiedliche Durchmesser haben, und/oder aufgrund der Tatsache, dass sie unterschiedliche Mengen einer lichtabsorbierenden Substanz ohne Fluoreszenzeigenschaft enthalten, eine unterschiedliche Transmission für Licht im blauen Wellenlängenbereich aufweisen, vorzugsweise von 0% bis zu 80%, und/oder aufgrund der Tatsache, dass sie unterschiedliche Mengen einer lichtabsorbierenden Substanz mit Fluoreszenzeigenschaft enthalten, grün, gelb, rot, infrarot oder weiß, leuchten, wenn sie mit dem Licht aus der Strahlungsquelle (1 1; 6c1 1; 21; 41; 61) angestrahlt werden, das seine Grundfarbe im sichtbaren Blau und/oder Violett hat.

## Claims

1. Appliance for dermatological or cosmetic treatment of a patient, with
a base body (19; 6c19), having a radiation source (11; 21; 41; 61; 6c11) for emission of light in a wavelength region between 320 nm and 1500 nm,
an intermediate piece (15; 25; 35; 45; 550; 551; 650; 6c15; 6c25), which is placed onto the base body (19; 6c19) in order to direct the light from the radiation source (11; 21; 41; 61; 6c11) toward the patient's body region (214) being treated, and
an attachment element (18; 28; 38; 414; 418; 58; 68; 6c18; 6d18; 6e18), arranged on the
light exit end of the intermediate piece (15; 25; 35; 45; 55; 550; 551; 650; 6c15; 6c25), which is designed to be brought into direct contact with the body region (214) being treated, which attachment element (18; 28; 38; 414; 418; 58; 68; 6c18; 6d18; 6e18) clamps the outer circumferential wall of the light exit end of the intermediate piece, and whereby the attachment element is deformable when pressed against the body region (214) being treated.
**characterized in that** the attachment element (18; 28; 38; 414; 418; 58; 68; 6c18; 6d18; 6e18) is embodied in the form of a cap containing an elastomer or a fluorinated thermoplastic material and is placed over the light exit end of the intermediate piece (15; 25; 35; 45; 550; 551; 650; 6c25), and that the attachment element (18; 28; 38; 414; 418; 58; 68; 6c18; 6d18; 6e18) consists entirely of the elastomer or the fluorinated thermoplastic material at least at its light exit surface.

2. Appliance according to claim 1, moreover with a base element (6c24; 6d24; 6e24) with cylindrical basic shape, which is detachably placed on a base of the intermediate piece (15; 25; 35; 45; 6c15; 6c25),
wherein a rigid or flexible light guide arrangement (550; 650) or the attachment element (6c18; 6d18; 6e18) is firmly joined to the base element (6c24; 6d24; 6e24), for example, by gluing.

3. Appliance according to claim 2, wherein the base element (6e24) has a first section for placing it with clamping fit on the base of the intermediate piece (15; 25; 35; 45; 6c15; 6c25) and a second section (6e24a) for fastening or gluing the light guide arrangement (550; 650) or the attachment element (6e18).

4. Appliance according to one of the preceding claims, wherein the elastomer has a Shore A hardness of less than 100, preferably between 30 and 70, especially around 45.

5. Appliance according to one of the preceding claims, wherein at least the light exit surface of the attachment element (18; 28; 38; 414; 418; 58; 68; 6c18; 6d18; 6e18) consists of a highly transparent material, especially an addition-linked silicone elastomer.

6. Appliance according to one of the preceding claims, wherein the attachment element (18; 28; 38; 414; 418; 58; 68; 6c18; 6d18; 6e18) contains a light absorbing substance without fluorescent property, especially carbon or graphite.

7. Appliance according to claim 6, wherein the attachment element (18; 28; 38; 414; 418; 58; 68; 6c18; 6d18; 6e18) contains 0.01 to 3 wt. % of the light absorbing substance without fluorescent property.

8. Appliance according to claim 6 or 7, wherein the light absorbing substance without fluorescent property absorbs between 10% and almost 100% of the light emitted by the radiation source (11; 21; 41; 61; 6c11) and converts it into heat.

9. Appliance according to one of the preceding claims, wherein the elastomer is chosen from one of the following materials: synthetic rubber, silicone rubber, especially addition-linked silicone rubber, polyurethane or polyethylene.

10. Appliance according to one of the preceding claims, wherein the fluorinated thermoplastic material is chosen from one of the following materials: fluorocarbon polymers such as Teflon® FEP, Teflon® MFA, Teflon® PTFE, Hyflon® THV.

11. Appliance according to one of the preceding claims, wherein the attachment element (18; 28; 38; 414; 418; 58; 68; 6c18; 6d18; 6e18) has the shape of a cap, whose tissue pressing surface (18a) can vary between a convex and a concave configuration.

12. Appliance according to one of the preceding claims, wherein the attachment element (18; 28; 38; 414; 418; 58; 68; 6c18; 6d18; 6e18) contains a light absorbing substance with fluorescent property, especially a phosphor based on perylene phosphors, particularly Lumogen®, or rare earths.

13. Appliance according to claim 12, wherein the attachment element (18; 28; 38; 414; 418; 58; 68; 6c18; 6d18; 6e18) doped with the light absorbing substance is coated with a thin layer of undoped silicone at least on its tissue pressing surface (18a).

14. Appliance according to one of the preceding claims, moreover with a mechanism (6c20, 6c21) designed to interrupt or reduce the voltage supply of the radiation source (6c11) when the pressing force of the attachment element (6c18; 6d18; 6e18) against the body region (214) being treated lies below a predetermined threshold value, and restore or increase the voltage supply of the radiation source (6c11) when the pressing force of the attachment element (6c18; 6d18; 6e18) against the body region (214) being treated lies above the predetermined threshold value.

15. Appliance according to claim 14, wherein a base of the intermediate piece (6c15; 6c22; 6c23; 6c25) has a connection body (6c15) firmly fastened to the base body (6c19) and a connection body (6c25) mounted movable in relation to the fixed connection body (6c15).

16. Appliance according to claim 15, wherein the mechanism (6c20, 6c21) has a switch element (6c20), which is arranged between the movable connection body (6c25) and the base body (6c19) and designed to be switched by the movable connection body (6c25) at a pressing force of the attachment element (6c18; 6d18; 6e18) against the body region (214) being treated beyond the predetermined threshold value to a state wherein the radiation source (6c11) is connected to the voltage supply or the voltage supply of the radiation source (6c11) is increased.

17. Appliance according to claim 16, wherein the mechanism (6c20, 6c21) moreover has an elastic element (6c21), which is arranged between the movable connection body (6c25) and the base body (6c19) and designed to force the movable connection body (6c25) away from the base body (6c19) and thereby switch the switch element (6c20) to a state wherein it interrupts the electrical connection of the radiation source (6c11) to the voltage source when the pressing force of the attachment element (6c18; 6d18; 6e18) against the body region (214) being treated lies below the predetermined threshold value, and the elastic element (6c21) is in particular a spring, such as a spiral spring.

18. Appliance according to one of the preceding claims, wherein the radiation source (11; 21; 41; 61; 6c11) is an LED or an LED array, whose total electric power is preferably between 5 and 25 W.

19. Appliance according to one of the preceding claims, wherein the radiation source (11; 21; 41; 61; 6c11) emits light in the wavelength region of 320 nm < λ < 1500 nm and preferably between 350 nm and 485 nm.

20. Appliance according to one of the preceding claims, designed to introduce a dermatologically or cosmetically active substance (213) into the body region (214) being treated under irradiation with the emitted light.

21. A system with an appliance according to one of the preceding claims and
at least one additional attachment element, suitable for replacing the attachment element (18; 28; 38; 414; 418; 58; 68; 6c18; 6d18; 6e18) and differing from it only in terms of size and light transmission properties, and/or at least one light guide arrangement (550; 650) for interchangeable placement on a base of the intermediate piece (15; 25; 35; 45; 6c15; 6c25).

22. System according to claim 21, wherein the attachment elements have different diameter, and/or
due to the fact that they contain different amounts of a light absorbing substance without fluorescing property, they have a different transmission for light in the blue wavelength region, preferably from 0% to 80%, and/or
due to the fact that they contain different amounts of a light absorbing substance with fluorescing property, they shine green, yellow, red, infrared or white when exposed to the light from the radiation source (11; 21; 41; 61; 6c11) having its basic color in the visible blue and/or violet.

## Revendications

1. Dispositif de traitement dermatologique ou cosmétique d'un patient, comortant un corps de base (19 ; 6c19), qui présente une source de rayonnement (11 ; 21 ; 41 ; 61 ; 6c11) pour une émission de lumière dans une plage de longueur d'onde entre 320 nm et 1 500 nm, une pièce intermédiaire (15; 25; 35; 45; 550; 551 ; 650 ; 6c15, 6c25), qui est appliquée sur le corps de base (19; 6c19) pour diriger la lumière depuis la source de rayonnement (11 ; 21 ; 41 ; 61 ; 6c11) en direction d'une zone corporelle (214) du patient à traiter, et un élément chapeau (18 ; 28 ; 38 ; 414 ; 418 ; 58 ; 68 ; 6c18 ; 6d18 ; 6e18), qui est disposé à l'extrémité de sortie de lumière de la pièce intermédiaire (15 ; 25 ; 35 ; 45 ; 55 ; 550 ; 650 ; 6c15, 6c25) et est conçu pour être amené en contact direct avec la zone corporelle (214) à traiter, l'élément chapeau (18 ; 28 ; 38 ; 414 ; 418 ; 58 ; 68 ; 6c18) siégeant à force sur la paroi périphérique extérieure de l'extrémité de sortie de lumière de la pièce intermédiaire et l'élément chapeau étant déformable sous la compression contre la zone corporelle (214) à traiter, **caractérisé en ce que** l'élément chapeau (18 ; 28 ; 38 ; 414 ; 418 ; 58 ; 68 ; 6c18 ; 6d8 ; 6e8) est configuré sous la forme d'un capuchon contenant un élastomère ou une matière plastique thermoplastique fluorée et étant coiffé sur l'extrémité de sortie de lumière de la pièce intermédiaire (15 ; 25 ; 35 ; 45 ; 550 ; 551 ; 650 ; 6c25), et **en ce que** l'élément chapeau (18; 28 ; 38 ; 414 ; 418 ; 58 ; 68 ; 6c18, 6d18 ; 6e18) est, au moins sur sa surface de sortie de lumière, composé entièrement de l'élastomère ou de la matière plastique thermoplastique fluorée.

2. Dispositif selon la revendication 1, comprenant en outre un élément socle (6c24 ; 6d24 ; 6e24) de forme de base cylindrique, qui est enfiché de manière amovible sur un corps de base de la pièce intermédiaire (15 ; 25 ; 35 ; 45 ; 6c15, 6c25), dans lequel un agencement de fibres optiques rigide ou flexible (550 ; 650) ou l'élément chapeau (6c18 ; 6d18 ; 6e18) est relié de manière solidaire à l'élément socle (6c24 ; 6d24 ; 6e24) par exemple par collage.

3. Dispositif selon la revendication 2, dans lequel l'élément socle (6e24) présente une première partie pour un enfichage par montage à force sur le corps de base de la pièce intermédiaire (15 ; 25 ; 35 ; 45 ; 6c15, 6c25) et une seconde partie (6e24a) pour fixer ou coller l'agencement de fibres optiques (550 ; 650) ou l'élément chapeau (6e8).

4. Dispositif selon une des revendications précédentes, dans lequel l'élastomère a une dureté Shore-A inférieure à 100, de préférence entre 30 et 70, en particulier d'environ 45.

5. Dispositif selon une des revendications précédentes, dans lequel au moins la surface de sortie de lumière de l'élément chapeau (18 ; 28 ; 38 ; 414 ; 418 ; 58 ; 68 ; 6c18 ; 6d18 ; 6e18) est composée d'un matériau à transparence élevée, en particulier d'un élastomère de silicone réticulé par addition.

6. Dispositif selon une des revendications précédentes, dans lequel l'élément chapeau (18 ; 28 ; 38 ; 414 ; 418 ; 58 ; 68 ; 6c18 ; 6d18 ; 6e18) contient une substance absorbant la lumière sans propriétés de fluorescence, en particulier du charbon ou du graphite.

7. Dispositif selon la revendication 6, dans lequel l'élément chapeau (18 ; 28 ; 38 ; 414 ; 418 ; 58 ; 68 ; 6c18 ; 6d18 ; 6e18) contient de 0,01 à 3 % en poids de la substance absorbant la lumière sans propriétés de fluorescence.

8. Dispositif selon la revendication 6 ou 7, dans lequel la substance absorbant la lumière sans propriétés de fluorescence absorbe entre 10 % et presque 100 % de la lumière émise par la source de rayonnement (11 ; 21 ; 41 ; 61 ; 6c11) et la transforme en chaleur.

9. Dispositif selon une des revendications précédentes, dans lequel l'élastomère est choisi parmi l'un des matériaux suivants : caoutchouc synthétique, caoutchouc silicone, en particulier caoutchouc silicone réticulé par addition, polyuréthanne ou polyéthylène.

10. Dispositif selon une des revendications précédentes, dans lequel la matière plastique thermoplastique fluorée est choisie parmi l'une des matières suivantes : polymères de carbone-fluor tels que le Téflon<®>FEP, le Téflon® MFA, le Téflon<®>PTFE, le Hyflon<®>THV.

11. Dispositif selon une des revendications précédentes, dans lequel l'élément chapeau (18 ; 28 ; 38 ; 414 ; 418 ; 58 ; 68 ; 6c18 ; 6d18 ; 6e18) a la forme d'un capuchon dont la surface de compression de tissu (18a) est variable entre une configuration convexe et une configuration concave.

12. Dispositif selon une des revendications précédentes, dans lequel l'élément chapeau (18 ; 28 ; 38 ; 414 ; 418 ; 58 ; 68 ; 6c18 ; 6d18 ; 6e18) contient une substance absorbant la lumière avec propriétés de fluorescence, en particulier des substances luminescentes à base de substances luminescentes pérylène, en particulier Lumogen®, ou des terres rares,.

13. Dispositif selon la revendication 12, dans lequel l'élément chapeau (18, 28, 38, 414, 418, 58, 68 ; 6c18 ; 6d18 ; 6e18) doté de la substance absorbant la lumière est, au moins sur sa surface de compression de tissu (18a), recouvert d'une mince couche de silicone non doté.

14. Dispositif selon une des revendications précédentes, comprenant en outre un mécanisme (6c20 ; 6c21), qui est conçu pour interrompre ou diminuer l'alimentation en tension de la source de rayonnement (6c11), lorsque la force de compression de l'élément chapeau (6c18 ; 6d18 ; 6e8) contre la zone corporelle (214) à traiter est en dessous d'une valeur seuil prédéfinie, et pour établir ou pour augmenter l'alimentation en tension de la source de rayonnement (6c11), lorsque la force de compression de l'élément chapeau (6c18 ; 6d18 ; 6e18) contre la zone corporelle (214) à traiter est au-dessus de la valeur seuil prédéfinie.

15. Dispositif selon la revendication 14, dans lequel un corps de+ base de la pièce intermédiaire (6c15, 6c22, 6c23, 6c25) présente un corps de liaison fixe (6c15) fixé de manière solidaire au corps de base (6c19) et un corps de liaison (6c25) monté mobile par rapport au corps de liaison fixe (6c15).

16. Dispositif selon la revendication 15, dans lequel le mécanisme (6c20, 6c21) présente un élément de commutation (6c20) qui est disposé entre le corps de liaison mobile (6c25) et le corps de base (6c19) et conçu pour être commuté par le corps de liaison mobile (6c25), lorsque la force de compression de l'élément chapeau (6c18 ; 6d18 ; 6e18) contre la zone corporelle (214) à traiter dépasse la valeur seuil prédéfinie, dans un état dans lequel la source de rayonnement (6c11) est raccordée à l'alimentation en tension ou dans lequel l'alimentation en tension de la source de rayonnement (6c11) est augmentée.

17. Dispositif selon la revendication 16, dans lequel le mécanisme (6c20, 6c21) présente en outre un élément élastique (6c21) qui est disposé entre le corps de liaison mobile (6c25) et le corps de base (6c19) et conçu pour pousser le corps de liaison mobile (6c25) à distance du corps de base (6c19) et commuter ainsi l'élément de commutation (6c20) dans un état dans lequel il interrompt la liaison électrique de la source de rayonnement (6c11) avec la source de tension lorsque la force de compression de l'élément chapeau (6c18 ; 6d18 ; 6e18) contre la zone corporelle (214) à traiter est en dessous de la valeur seuil prédéfinie, et l'élément élastique (6c21) est en particulier un ressort, par exemple un ressort en spirale.

18. Dispositif selon une des revendications précédentes, dans lequel la source de rayonnement (11 ; 21 ; 41 ; 61 ; 6c11) présente une LED ou un ensemble de LED dont la puissance électrique totale est de préférence comprise entre 5 et 25 watts.

19. Dispositif selon une des revendications précédentes, dans lequel la source de rayonnement (11 ; 21 ; 41 ; 61 ; 6c11) émet une lumière dans la plage de longueur d'onde 320 nm < λ < 1 500 nm et de préférence entre 350 nm et 485 nm.

20. Dispositif selon une des revendications précédentes, conçu pour apporter une substance dermatologique ou cosmétique efficace (213) par irradiation de la lumière émise dans la zone corporelle (214) à traiter.

21. Système comprenant un dispositif selon une des revendications précédentes et au moins un autre élément chapeau, qui est adapté pour remplacer l'élément chapeau (18 ; 28 ; 38 ; 414 ; 418 ; 58 ; 68 ; 6c18 ; 6d18 ; 6e18) et se distingue de celui-ci uniquement en termes de dimensions et de propriétés de transmission lumineuse, et/ou au moins un agencement de conduit de lumière (550 ; 650) pour un enfichage de manière échangeable sur un corps de base de la pièce intermédiaire (15 ; 25 ; 35 ; 45 ; 6c5, 6c25).

22. Système selon la revendication 21, dans lequel les éléments chapeaux ont des diamètres différents, et/ou, du fait qu'ils contiennent des quantités différentes d'une substance absorbant la lumière sans propriétés de fluorescence, présentent une transmission différente pour la lumière dans la plage de longueur d'onde bleue, de préférence de 0 % à 80 %, et/ou du fait qu'ils contiennent des quantités différentes d'une substance absorbant la lumière avec des propriétés de fluorescence, éclairent vert, jaune, rouge, infrarouge, ou blanc lorsqu'ils sont illuminés avec la lumière provenant de la source de rayonnement (11 ; 6c11 ; 21 ; 41 ; 61), qui a sa couleur de base dans le bleu et/ou violet visible.
